# EUROPEAN PATENT APPLICATION

(11) **EP 2 636 662 A1**
(43) Date of publication of application: **11.09.2013**
(21) Application number: 11839315.6
(22) Date of filing: 27.09.2011
(51) Int. Cl.: C07C 17/20, B01J 27/132, C07C 17/38, C07C 21/18, C07B 61/00

(54) **PROCESS FOR PRODUCING TRANS-1,3,3,3-TETRAFLUOROPROPENE**

(30) Priority: 15.09.2011 JP 2011202071; 10.11.2010 JP 2010251960
(71) Applicant: Central Glass Company, Limited, Yamaguchi 755-0001 (JP)
(72) Inventor: HIBINO, Yasuo, Kawagoe-shi, Saitama 350-1159 (JP); YOSHIKAWA, Satoshi, Kawagoe-shi, Saitama 350-1159 (JP); NISHIGUCHI, Yoshio, Kawagoe-shi, Saitama 350-1159 (JP); OKAMOTO, Satoru, Kawagoe-shi, Saitama 350-1159 (JP); SAKYU, Fuyuhiko, Kawagoe-shi, Saitama 350-1159 (JP)
(74) Representative: Manitz, Finsterwald & Partner GbR
(86) International application number: PCT/JP2011/071947
(87) International publication number: WO 2012/063566

(57) **Abstract**

A production process of trans-1,3,3,3-tetrafluoropropene according to the present invention includes: a reaction step of reacting 1-chloro-3,3,3-trifluoropropene with hydrogen fluoride to form trans-1,3,3,3-tetrafluoropropene and obtain a reaction product A containing the formed trans-1,3,3,3-tetrafluoropropene, unreacted 1-chloro-3,3,3-trifloropropene and hydrogen fluoride and by-produced cis-1,3,3,3-tetrafluoropropene, 1,1,1,3,3-pentafluoropropane and hydrogen chloride; a rough separation step of distilling the reaction product A obtained in the reaction step to recover a distillation bottom product containing the 1-chloro-3,3,3-trifloropropene and hydrogen fluoride, and then, supplying the recovered distillation bottom product to the reaction step; a hydrogen fluoride separation step of recovering the hydrogen fluoride from a residue B remaining after the recovery of the distillation bottom product in the rough separation step and supplying the recovered hydrogen fluoride to the reaction step; a hydrogen chloride separation step of bringing a residue C remaining after the recovery of the hydrogen fluoride in the hydrogen fluoride separation step into contact with water or an aqueous sodium hydroxide solution to thereby separate the hydrogen chloride; a dehydration drying step of dehydrating a residue D remaining after the separation of the hydrogen chloride in the hydrogen chloride separation step; and a purification step of obtaining the trans-1,3,3,3-tetrafluoropropene by distillation of a residue E remaining after the dehydration in the dehydration drying step. It is possible in the present production process to reuse the unreacted reactants and produce the target compound efficiently.

## Description

### Field of the Invention

The present invention relates to a process for production of trans-1,3,3,3-tetrafluoropropene, which is useful as an intermediate raw material for pharmaceutical and agrichemical products and functional materials, a propellant for aerosols such as a spray, a protection gas for production of magnesium alloys, a blowing agent, an extinguishing agent, a semiconductor gas such as an etching gas, a heating medium, a cooling medium and the like.

### Background Art

The following processes are known as methods for production of 1,3,3,3-tetrafluoropropene.

For example, Non-Patent Document 1 discloses a process of dehydroiodination of 1,3,3,3-tetrafluoro-1-iodopropane with alcoholic potassium hydroxide.

Non-Patent Document 2 discloses a process of dehydrofluorination of 1,1,1,3,3-pentafluoropropane with potassium hydroxide in dibutyl ether.

Both of the processes of Non-Patent Documents 1 and 2, which involve dehydrohalogenation with potassium hydroxide, can attain high reaction rate and high selectivity of 1,3,3,3-tetrafluoropropene. However, each of these processes needs to use the solvent and to use the potassium hydroxide in a required stoichiometric amount or more and gives an enormous amount of potassium salt as a by-product. It is thus difficult, due to poor operability and high cost etc., to adopt the processes of Non-Patent Documents 1 and 2 as production methods in industrial plants for commercial production.

Patent Document 1 discloses a process of dehydrofluorination of 1,1,1,3,3-pentafluoropropane with the use of a metal compound such as chromium compound supported on activated carbon as a catalyst.

Patent Document 2 discloses a process of contact of 1,1,1,3,3-pentafluoropropane with a chromium-based catalyst.

Patent Document 3 discloses a process for production of 1,3,3,3-tetrafluoropropene by dehydrofluorination of 1,1,1,3,3-pentafluoropropane in a gas phase in the presence of a catalyst, wherein the catalyst is a supported zirconium-compound catalyst having a zirconium compound supported on a metal oxide or activated carbon.

Patent Document 4 discloses a process of reacting 1-chloro-3,3,3-trifluoropropene with hydrogen fluoride in a gas phase in the presence of a fluorination catalyst.

In the process for production of 1,3,3,3-tetrafluoropropene as disclosed in Patent Document 4, however, there is a problem that the selectivity of the 1,3,3,3-tetrafluoropropene is low. More specifically, the process of Patent Document 4 gives not only the 1,3,3,3-tetrafluoropropene but also a highly fluorinated by-product, that is, 1,1,1,3,3-pentafluoropropane due to further progress of fluorination so that the selectivity of the 1,3,3,3-tetrafluoropropene becomes decreased.

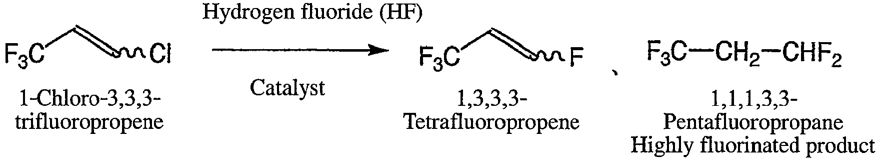

Patent Document 5 discloses a process of reacting 1,1,1,3,3-pentachloropropane with hydrogen fluoride in a gas phase in the presence of a fluorination catalyst.

Patent Document 6 discloses a process for production of 1,3,3,3-tetrafluoropropene, including: a first step of forming 1,1,1-trifluoro-3-chloro-2-propene (1-chloro-3,3,3-trifluoropropene) predominantly by reaction of 1,1,1,3,3-pentachloropropane with hydrogen fluoride in a gas phase; and a second step of, after removing hydrogen chloride from the gaseous product of the first step, reacting the gaseous product with hydrogen fluoride in a gas phase to form 1,1,1,3,3-pentafluoropropane. In the production process of Patent Document 6, the 1,1,1,3,3-pentachloropropane is used as the raw material and converted to the 1,3,3,3-tetrafluoropropene through the above two steps. This enables efficient production of the target 1,3,3,3-tetrafluoropropene as the conversion rate of the raw material and the selectivity of the target 1,3,3,3-tetrafluoropropene can be increased to significantly reduce the amount of the unreacted raw material or unsaturated intermediate product difficult to separate by distillation from the reaction product.

In the process for production of 1,3,3,3-tetrafluoropropene as disclosed in Patent Document 6, however, there still remains a problem that the selectivity of the 1,3,3,3-tetrafluoropropene is low. More specifically, although the process of Patent Document 6 gives a mixture of hydrogen chloride, 1,3,3,3-tetrafluoropropene, 1,1,1,3,3-pentafluoropropane, unreacted 1-chloro-3,3,3-trifluoropropene and unreacted hydrogen fluoride as the reaction product of the second step, it is seen from the comparison of the yields of 1,3,3,3-tetrafluoropropene and 1,1,1,3,3-pentafluoropropane that the selectivity of the 1,3,3,3-tetrafluoropropene is low.

Patent Document 7 discloses a process for production of 1,3,3,3-tetrafluoropropene, including: a first step of forming 1-chloro-3,3,3-trifluoropropene by reaction of 1,1,1,3,3-pentachloropropane with hydrogen fluoride; and a second step of forming 1,3,3,3-tetrafluoropropene by reaction of the 1-chloro-3,3,3-trifluoropropene obtained in the first step with hydrogen fluoride in a gas phase in the presence of a fluorination catalyst as indicated in the following scheme. 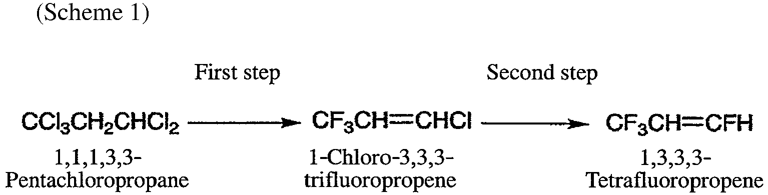 In Patent Document 7, the fluorination catalyst of the second step is either activated carbon, activated carbon supporting thereon an oxide, a fluoride, a chloride, a fluorochloride, an oxyfluoride, an oxychloride or oxyfluorochloride of at least one kind of metal or two or more kinds of metals selected from chromium, titanium, aluminum, manganese, nickel, cobalt and zirconium, alumina, fluorinated alumina, aluminum fluoride, zirconia or fluorinated zirconia.

Further, there are disclosed the following processes for production of 1,3,3,3-tetrafluoropropene through the use of catalysts such as antimony compounds.

Patent Document 8 discloses a process of liquid-phase fluorination of 1,1,1,3,3-pentafluoropropane with hydrogen fluoride in the presence of an antimony catalyst.

Patent Document 9 discloses a process of liquid-phase fluorination of 1-chloro-3,3,3-trifluoropropene with hydrogen fluoride in the presence of an antimony catalyst.

Patent Document 10 discloses a process for producing 1,1,1,3,3-pentafluoropropane by liquid-phase fluorination of 1,1,1,3,3-pentachloropropane with hydrogen fluoride in the presence of an antimony catalyst, wherein the 1,1,1,3,3-pentachloropropane and hydrogen fluoride are continuously supplied into the reaction zone.

Patent Document 11 discloses a process of addition of hydrogen fluoride to 1,3,3,3-tetrafluoropropene in the presence of a halide of one kind of metal or two or more kinds of metals selected from aluminum, tin, bismuth, antimony and iron as a hydrogen fluoride addition catalyst.

Patent Document 12 discloses a process of formation of 1,1,1,3-tetrafluoro-3-chloropropane by addition of hydrogen fluoride to 1-chloro-3,3,3-trifluoropropene in the presence of an addition catalyst, followed by disproportionation of the 1,1,1,3-tetrafluoro-3-chloropropane in the presence of a disproportionation catalyst.

Patent Document 13 discloses a process for production of 1,1,1,3,3-pentafluoropropane from 1-chloro-3,3,3-trifluoropropene and hydrogen fluoride in the presence of chlorine, wherein the reaction is performed by the use of a fluorination reaction apparatus having reactors (A) and (B) each packed with activated carbon supporting thereon antimony pentachloride and, more specifically, by replacing the reactors (A) and (B) repeatedly to select the reactors (A) and (B) as the first and second reactors, respectively, during a first time period and select the reactors (A) and (B) as the second and first reactors, respectively, during a second time period with the proviso that the first reactor whose setting temperature is 150°C or higher and the second reactor whose setting temperature is 20 to 150°C are arranged in line from the upstream side.

### Prior Art Documents

### Patent Documents

Patent Document 1: Japanese Laid-Open Patent Publication No. H11-140002
Patent Document 2: Japanese Laid-Open Patent Publication No. 2000-63300
Patent Document 3: Japanese Laid-Open Patent Publication No. 2008-19243
Patent Document 4: Japanese Laid-Open Patent Publication No. H10-7604
Patent Document 5: Japanese Laid-Open Patent Publication No. H10-7605
Patent Document 6: Japanese Laid-Open Patent Publication No. H9-183740
Patent Document 7: Japanese Laid-Open Patent Publication No. 2010-100613
Patent Document 8: Japanese Laid-Open Patent Publication No. H8-239334
Patent Document 9: Japanese Laid-Open Patent Publication No. H9-241188
Patent Document 10: Japanese Laid-Open Patent Publication No. H9-268141
Patent Document 11: Japanese Laid-Open Patent Publication No. H10-17502
Patent Document 12: Japanese Laid-Open Patent Publication No. H10-72381
Patent Document 13: Japanese Laid-Open Patent Publication No. 2002-105006

### Non-Patent Documents

Non-Patent Document 1: R.N.Haszeldine et al., J.Chem.Soc.1953, 1199-1206; CA 48 5787f
Non-Patent Document 2: I.L.Knunyants et al., Izvest.Akad.Nauk S.S.R., Otdel.Khim.Nauk. 1960,1412-18;CA 55, 349f

### Summary of the Invention

### Problems to be Solved by the Invention

As a result of extensive researches, the present inventors have found the process for production of 1,3,3,3-tetrafluoropropene as disclosed in Patent Document 7 to be superior but have also found that it is important in this process to recover unreacted hydrogen fluoride and, when the unreacted hydrogen fluoride is not recovered, is difficult to extract the trans-1,3,3,3-tetrafluoropropene in the subsequent distillation step. In the process of Patent Document 7, the reaction of the second step has a chemical equilibrium. It is necessary to increase the stoichiometric ratio of the hydrogen fluoride relative to the 1-chloro-3,3,3-trifluoropropene in the raw material and to control the reaction temperature to within a suitable range in order to shift the chemical equilibrium to the target 1,3,3,3-tetrafluoropropene side for high-yield production of the 1,3,3,3-tetrafluoropropene. The yield of the 1,3,3,3-tetrafluoropropene and the life of the fluorination catalyst before deactivation in the suitable reaction range become decreased unless the hydrogen fluoride is added in an excessive amount to the 1-chloro-3,3,3-trifluoropropene. The 1,3,3,3-tetrafluoropropene can be obtained efficiently as the selectivity of the 1,3,3,3-tetrafluoropropene becomes increased as the result of reacting the hydrogen fluoride at high concentration with the 1-chloro-3,3,3-trifluoropropene. Further, it is very difficult to separate an acidic by-product component such as hydrogen chloride by water washing in the subsequent step unless the hydrogen fluoride is separated and recovered from the reaction product.

As mentioned above, the conventional 1,3,3,3-tetrafluoropropene production processes have the problems that: it is difficult due to poor operability and high cost etc. to adopt in an industrial plant for commercial production: and the selectivity and yield of the 1,3,3,3-tetrafluoropropene in the formation reaction of the 1,3,3,3-tetrafluoropropene is low.

It is accordingly an object of the present invention to solve the above-mentioned prior art problems and to provide a process for producing trans-1,3,3,3-tetrafluoropropene with high efficiency and high yield on an industrial scale in an industrial plant for commercial production use by increasing the selectivity and yield of the trans-1,3,3,3-tetrafluoropropene in the formation reaction of the trans-1,3,3,3-tetrafluoropropene and purifying the trans-1,3,3,3-tetrafluoropropene to high purity by separation. It is also an object of the present invention to provide a process for producing trans-1,3,3,3-tetrafluoropropene highly efficiently in an energy-conserving and environmentally-friendly manner by reuse of unreacted reactants and minimization of by-products.

### Means for Solving the Problems

The present inventors have made extensive researches to solve the above-mentioned problems and, as a result, have found that it is possible to obtain trans-1,3,3,3-tetrafluoropropene with high selectivity and high yield by, at the time of forming the trans-1,3,3,3-tetrafluoropropene as a target product compound by reaction of 1-chloro-3,3,3-trifluoropropene and hydrogen fluoride as raw reactants, recovering unreacted 1-chloro-3,3,3-trifluoropropene and hydrogen fluoride from a mixture of the target product compound and by-products (hereinafter referred to as "reaction product" or "residue") and returning the recovered 1-chloro-3,3,3-trifluoropropene and hydrogen fluoride as the reactants into the reaction system. The present inventors have further found a technique for purifying the trans-1,3,3,3-tetrafluoropropene efficiently to high purity by separation. Based on these findings, the present inventors have established a process for producing trans-1,3,3,3-tetrafluoropropene efficiently with less by-products.

In the present specification, the term "reaction product" refers to a mixture resulting from the reaction that contains trans-1,3,3,3-tetrafluoropropene as a target compound, 1-chloro-3,3,3-trifluoropropene and hydrogen fluoride as unreacted reactants and by-products such as hydrogen chloride and other organic compounds. The term "operation" refers to a treatment for, for example, removing hydrogen fluoride, removing hydrogen chloride, removing water or removing any organic compounds other than the target compound, that is, 1,3,3,3-tetrafluoropropene by distillation to recover those organic compounds as a distillation bottom product. In contrast to the reaction product, the term "residue" refers to any substance remaining after the removal of the target matter by the operation.

Further, the term "1-chloro-3,3,3-trifluoropropene" refers to a mixture of cis and trans isomers thereof unless otherwise specified in the present specification. Similarly, the term "1,3,3,3-tetrafluoropropene" refers to a mixture of cis and trans isomers thereof. The trans-1-chloro-3,3,3-trifluoropropene has a boiling point of 21°C; and the cis-1-chloro-3,3,3-trifluoropropene has a boiling point of 39°C. The trans-1,3,3,3-tetrafluoropropene has a boiling point of -19°C; and the cis-1,3,3,3-tetrafluoropropene has a boiling point of 9°C. Further, 1,1,1,3,3-pentafluoropropane has a boiling point of 15°C. It is possible to separate the trans-1,3,3,3-tetrafluoropropene from the mixture of these compounds by distillation due to differences in boiling points. The selectivity of the trans-1,3,3,3-tetrafluoropropene refers to the proportion of the trans-1,3,3,3-tetrafluoropropene in the reaction product obtained by conversion of the 1-chloro-3,3,3-trifluoropropene. The reaction yield, just referred to as yield, of the trans-1,3,3,3-tetrafluoropropene can be determined by multiplication of the conversion rate of the 1-chloro-3,3,3-trifluoropropene as the raw reactant material by the selectivity of the trans-1,3,3,3-tetrafluoropropene.

In other words, the present invention includes the following inventive aspects 1 to 13.

### [Inventive Aspect 1]

A process for production of trans-1,3,3,3-tetrafluoropropene, comprising:
a reaction step of reacting 1-chloro-3,3,3-trifluoropropene with hydrogen fluoride to form trans-1,3,3,3-tetrafluoropropene and obtain a reaction product A containing the formed trans-1,3,3,3-tetrafluoropropene, unreacted 1-chloro-3,3,3-trifloropropene and hydrogen fluoride and by-produced cis-1,3,3,3-tetrafluoropropene, 1,1,1,3,3-pentafluoropropane and hydrogen chloride;
a rough separation step of distilling the reaction product A obtained in the reaction step to recover a distillation bottom product containing the 1-chloro-3,3,3-trifloropropene and hydrogen fluoride, and then, supplying the recovered distillation bottom product to the reaction step;
a hydrogen fluoride separation step of recovering the hydrogen fluoride from a residue B remaining after the recovery of the distillation bottom product in the rough separation step and supplying the recovered hydrogen fluoride to the reaction step;
a hydrogen chloride separation step of bringing a residue C remaining after the recovery of the hydrogen fluoride in the hydrogen fluoride separation step into contact with water or an aqueous sodium hydroxide solution to thereby separate the hydrogen chloride;
a dehydration drying step of dehydrating a residue D remaining after the separation of the hydrogen chloride in the hydrogen chloride separation step; and
a purification step of obtaining the trans-1,3,3,3-tetrafluoropropene by distillation of a residue E remaining after the dehydration in the dehydration drying step.

### [Inventive Aspect 2]

The process according to Inventive Aspect 1, wherein, in the reaction step, the trans-1,3,3,3-tetrafluoropropene is formed by fluorination of the 1-chloro-3,3,3-trifluoropropene with the hydrogen chloride in a gas phase in the presence of a fluorination catalyst.

### [Inventive Aspect 3]

The process according to Inventive Aspect 2, wherein, in the reaction step, the fluorination is performed in the gas phase under the conditions of a pressure of 0.05 to 0.3 MPa and a temperature of 200 to 450°C.

### [Inventive Aspect 4]

The process according to Inventive Aspect 2 or 3, wherein the fluorination catalyst is either a nitrate, a chloride, an oxide, a sulfate, a fluoride, a fluorochloride, an oxyfluoride, an oxychloride or an oxyfluorochloride of at least one kind of metal selected from the group consisting of chromium, titanium, aluminum, manganese, nickel, cobalt, titanium, iron, copper, zinc, silver, molybdenum, zirconium, niobium, tantalum, iridium, tin, hafnium, vanadium, magnesium, lithium, sodium, potassium, calcium and antimony.

### [Inventive Aspect 5]

The process according to Inventive Aspects 1 to 3, wherein, in the reaction step, the fluorination is performed in the gas phase in the presence of chromium chloride supported on fluorinated alumina as the fluorination catalyst under the conditions of a pressure of 0.05 to 0.3 MPa and a temperature of 350 to 450°C by the supply of the 1-chloro-3,3,3-trifluoropropene and hydrogen fluoride at a mole ratio of 1-chloro-3,3,3-trifluoropropene:hydrogen fluoride = 1:8 to 1:25.

### [Inventive Aspect 6]

The process according to Inventive Aspects 1 to 3, wherein, in the reaction step, the fluorination is performed in the gas phase in the presence of either an oxide, a fluoride, a chloride, a fluorochloride, an oxyfluoride, an oxychloride or an oxyfluorochloride of chlromium supported on activated carbon as the fluorination catalyst under the conditions of a pressure of 0.05 to 0.3 MPa and a temperature of 350 to 450°C by the supply of the 1-chloro-3,3,3-trifluoropropene and hydrogen fluoride at a mole ratio of 1-chloro-3,3,3-trifluoropropene:hydrogen fluoride = 1:8 to 1:25.

### [Inventive Aspect 7]

The process according to Inventive Aspects 1 to 6, wherein, in the hydrogen fluoride separation step, the hydrogen fluoride is recovered by absorption into sulfuric acid.

### [Inventive Aspect 8]

The process according to Inventive Aspects 1 to 7, wherein, in the dehydration drying step, the residue D remaining after the hydrogen chloride separation step is dehydrated by freezing and solidifying water contained in the residue D by means of a heat exchanger.

### [Inventive Aspect 9]

The process according to Inventive Aspects 1 to 7, wherein, in the dehydration drying step, the residue D remaining after the hydrogen chloride separation step is dehydrated by adsorption of water contained in the residue D onto an adsorbent.

### [Inventive Aspect 10]

The process according to Inventive Aspects 1 to 10, further comprising a step of supplying a distillation residue F remaining after the purification step to the reaction step.

### [Inventive Aspect 11]

The process according to Inventive Aspect 10, wherein the distillation residue F remaining after the purification step is supplied to the reaction step after converting the cis-1,3,3,3-tetrafluoropropene contained in the distillation residue F to 1,1,1,3,3-pentafluoropropane.

As mentioned above, the production process of the present invention is environmentally friendly as the unreacted reactants, i.e., 1-chloro-3,3,3-trifluoropropene and hydrogen fluoride are recovered from the product of the reaction step and returned to and reused as the raw material in the reaction system of the reaction step. In addition, the present production process is high in productivity as the trans-1,3,3,3-tetrafluoropropene can be obtained with higher yield and higher purity than conventional production processes even under industrially practicable, easy production conditions. This results from: forming the target trans-1,3,3,3-tetrafluoropropene with high selectivity by using the easily available 1-chloro-3,3,3-trifluoropropene as the raw reactant material in the reaction step, selecting the specific fluorination catalyst for the reaction of the 1-chloro-3,3,3-trifluoropropene with the excessive amount of hydrogen fluoride and adjusting the reaction temperature to maintain the catalytic activity of the fluorination catalyst during the reaction; recovering the unreacted 1-chloro-3,3,3-trifluoropropene and hydrogen fluoride from the reaction product in the subsequent purification step and returning these unreacted reactants to the reaction step; and recovering the hydrogen fluoride from the reaction product in the subsequent fluorination separation step and returning the recovered hydrogen fluoride to the reaction step. By the series of these operations, the excessive amount of hydrogen fluoride can be easily supplied relative to the 1-chloro-3,3,3-trifluoropropene in the reaction step. Furthermore, the reaction product is subjected to water washing in the subsequent hydrogen chloride separation step to separate the by-produced hydrogen, and then, dehydrated in the subsequent dehydration drying step to remove water contained due to the water washing of the preceding hydrogen chloride separation step. This leads to a reduction in the load of distillation of the reaction product in the subsequent purification step so that the trans-1,3,3,3-tetrafluoropropene can be easily obtained with high purity.

### Brief Description of the Drawing

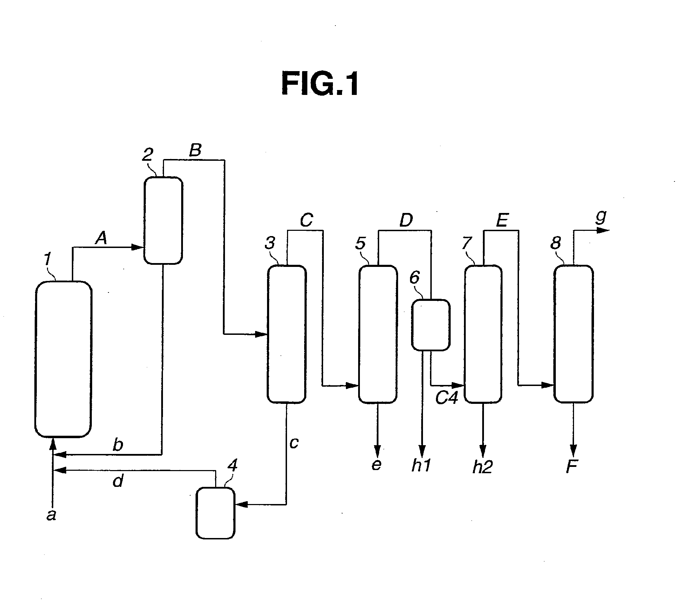

FIG. 1 is an example of a flow chart of a process for production of trans-1,3,3,3-tetrafluoropropene according to the present invention.

### Detailed Description of the Embodiments

The present invention will be described in detail below.

### 1. Production Process of Trans-1,3,3,3-tetrafluoropropene

A production process of trans-1,3,3,3-tetrafluoropropene according to the present invention includes the following steps:
a reaction step (first step) of reacting a raw material, i.e., 1-chloro-3,3,3-trifluoropropene with hydrogen fluoride within a reactor to form trans-1,3,3,3-tetrafluoropropene as a target compound and thereby obtain a reaction product A containing the formed trans-1,3,3,3-tetrafluoropropene, unreacted 1-chloro-3,3,3-trifloropropene and hydrogen fluoride and by-products such as cis-1,3,3,3-tetrafluoropropene, 1,1,1,3,3-pentafluoropropane and hydrogen chloride;
a rough separation step (second step) of distilling the reaction product A to recovering a distillation bottom product containing the 1-chloro-3,3,3-trifloropropene and hydrogen fluoride, and then, supplying the recovered distillation bottom product into the reactor of the reaction step;
a hydrogen fluoride separation step (third step) of recovering the hydrogen fluoride from a residue B remaining after the recovery of the distillation bottom product in the rough separation step and supplying the recovered hydrogen fluoride into the reactor of the reaction step;
a hydrogen chloride separation step (fourth step) of bringing a residue C remaining after the recovery of the hydrogen fluoride in the hydrogen fluoride separation step into contact with water or an aqueous sodium hydroxide solution to thereby separate the hydrogen chloride;
a dehydration drying step (fifth step) of dehydrating a residue D remaining after the separation of the hydrogen chloride in the hydrogen chloride separation step; and a purification step (sixth step) of obtaining the trans-1,3,3,3-tetrafluoropropene by distillation of a residue E remaining after the dehydration in the dehydration drying step.

The production process of the present invention is characterized by adopting, after the reaction step, the rough separation step in which the unreacted 1-chloro-3,3,3-trifluoropropene and hydrogen fluoride are recovered by distillation of the reaction product A as the distillation bottom product and returned to the reaction step and the hydrogen fluoride separation step in which the hydrogen fluoride is recovered from the residue B of the rough separation step and returned to the reaction step. Herein, the distillation operation of the rough separation step is occasionally referred to as rough distillation. By returning the recovered unreacted 1-chloro-3,3,3-trifluoropropene and hydrogen fluoride as the raw material to the reaction step, it is possible to easily supply the hydrogen fluoride in an excessive amount relative to the 1-chloro-3,3,3-trifluoropropene and obtain the target trans-1,3,3,3-tetrafluoropropene with high selectivity and high yield. It is further possible by recovering and removing the hydrogen fluoride from the reaction product A and from the residue B to significantly reduce the loads of the subsequent hydrogen fluoride separation step, dehydration drying step and purification steps (fourth to sixth steps) caused due to the existence of the unreacted hydrogen fluoride in the reaction product. It is also possible to easily and selectively separate and purify the trans-1,3,3,3-tetrafluoropropene to high purity by distillation of the residue E in the purification step and thereby significantly increase the efficiency of the purification operation. As mentioned above, the production process of the present invention allows efficient production of the target compound with less by-products by the effective use of the raw material and thus can be regarded as an environmentally friendly method suitable for industrial production.

In the reaction step, the formation reaction of trans-1,3,3,3-tetrafluoropropene from 1-chloro-3,3,3-trifluoropropene and hydrogen fluoride has a chemical equilibrium. In order to shift the chemical equilibrium to the target compound side, it is necessary to use the hydrogen fluoride in a stoichiometric amount or more relative to the 1-chloro-3,3,3-trifluoropropene as mentioned above. It is possible, by using such an excessive amount of hydrogen fluoride relative to 1-chloro-3,3,3-trifluoropropene as well as by selecting a specific fluorination catalyst and optimizing the reaction conditions (pressure, temperature and the like) for the fluorination catalyst, to protect the fluorination catalyst and maintain the catalytic activity of the fluorination catalyst so that the trans-1,3,3,3-tetrafluoropropene can be obtained with high selectivity and high yield.

As the reaction product (residue C) is subjected to water washing or brought into contact with the aqueous sodium hydroxide solution in order to separate the hydrogen chloride in the hydrogen chloride separation step, water is contained in the reaction product. However, the reaction product is dehydrated to remove such entrained water in the subsequent dehydration drying step. It is thus possible to easily purify the trans-1,3,3,3-tetrafluoropropene in the purification step.

The production process of the present invention may further include, after the extraction of the trans-1,3,3,3-tetrafluoropropene by distillation of the residue E in the purification step, a step of supplying a distillation residue containing the 1-chloro-3,3,3-trifluoropropene, cis-1,3,3,3-tetrafluoropropene and 1,1,1,3,3-pentafluoropropane (occasionally referred to as "residue F") to the reaction step. It is possible by this step to secure further supply of the unreacted reactant in addition to the effective use of the by-product. At this time, the trans-1,3,3,3-tetrafluoropropene can be obtained with higher efficiency by supplying the residue F to the reaction system after converting the cis-1,3,3,3-tetrafluoropropene in the residue F to 1,1,1,3,3-pentafluoropropane.

Thus, the production process of the present invention preferably includes the following operations (a) to (c):
operation (a) for continuously reacting the 1-chloro-3,3,3-trifluoropropene with the hydrogen fluoride in the presence of the fluorination catalyst to obtain the reaction product A containing the trans-1,3,3,3-tetrafluoropropene as the target compound, the unreacted 1-chloro-3,3,3-trifluoropropene and hydrogen fluoride and the by-products such as cis-1,3,3,3-tetrafluoropropene, 1,1,1,3,3-pentafluoropropane and hydrogen chloride (in this operation, it is particularly preferable to set the reaction temperature to 350 to 400°C and to supply the 1-chloro-3,3,3-trifluoropropene and hydrogen fluoride at a mole ratio of 1-chloro-3,3,3-trifluoropropene:hydrogen fluoride = 1:8 to 1:25);
operation (b) for separating the hydrogen fluoride, cis-1,3,3,3-tetrafluoropropene, 1,1,1,3,3-pentafluoropropane and 1-chloro-3,3,3-trifluoropropene from the reaction product A to recover the trans-1,3,3,3-tetrafluoropropene; and
operation (c) for supplying the separated hydrogen fluoride, cis-1,3,3,3-tetrafluoropropene, 1,1,1,3,3-pentafluoropropane and 1-chloro-3,3,3-trifluoropropene again to the operation (a) after converting the cis-1,3,3,3-tetrafluoropropene to 1,1,1,3,3-pentafluoropropane.

### 2. Process Steps

The respective steps of the production process of the present invention will be described in more detail below.

### 2.1. Reaction Step (First Step)

The reaction step (first step) will be first explained below.

The reaction step is a step of forming trans-1,3,3,3-tetrafluoropropene by reaction of 1-chloro-3,3,3-trifluoropropene with hydrogen fluoride in a gas phase in the presence of a fluorination catalyst to thereby obtain a reaction product A containing 1-chloro-3,3,3-trifluoropropene and hydrogen fluoride as unreacted substrates, trans-1,3,3,3-tetrafluoropropene as a target compound and by-products such as hydrogen chloride and other organic compounds.

It is preferable in the reaction step to supply the hydrogen fluoride in an excessive amount relative to the 1-chloro-3,3,3-trifluoropropene as the raw material into the reaction system and, more specifically, into the reactor in order to increase the selectivity and yield of the trans-1,3,3,3-tetrafluoropropene. The supply of such an excessive hydrogen fluoride also leads to protection of the fluorination catalyst so as to increase the catalytically active life of the fluorination catalyst. Herein, the 1-chloro-3,3,3-trifluoropropene used as the raw reactant material in the reaction step can exist as a cis isomer or a trans isomer. The reaction proceeds favorably regardless of whether the 1-chloro-3,3,3-trifluoropropene is the form of either a cis isomer, a trans isomer or a mixture thereof.

In the reaction step, a metal oxide, a fluorinated metal oxide or a metal salt can be used as the fluorination catalyst.

Examples of metals of the metal oxide, fluorinated metal oxide and metal salt are chromium, titanium, aluminum, manganese, nickel, cobalt, titanium, iron, copper, zinc, silver, molybdenum, zirconium, niobium, tantalum, iridium, tin, hafnium, vanadium, magnesium, lithium, sodium, potassium, calcium and antimony.

More specifically, the fluorination catalyst is preferably a nitrate, a chloride, an oxide, a sulfate, a fluoride, a fluorochloride, an oxyfluoride, an oxychloride or an oxyfluorochloride of at least one kind of metal selected from the group consisting of chromium, titanium, aluminum, manganese, nickel, cobalt, titanium, iron, copper, zinc, silver, molybdenum, zirconium, niobium, tantalum, iridium, tin, hafnium, vanadium, magnesium, lithium, sodium, potassium, calcium and antimony.

Preferably, the metal oxide has a part or all of oxygen atoms replaced with a fluorine atom by treatment with hydrogen fluoride or a fluorine-containing organic compound. There can be used fluorinated oxides each having a part or all of oxygen atoms replaced with a fluorine atom by fluorination of alumina, chromia, zirconia, titania, magnesia or the like. Among others, it is particularly preferable to use fluorinated alumina obtained by fluorination of activated alumina with hydrogen fluoride etc. The fluorinated metal oxide is hereinafter occasionally just referred to as "metal oxide" in the present invention.

It is feasible to use a commercially available metal oxide. It is also feasible to prepare a metal oxide by any known catalyst preparation technique and, more specifically, by e.g. controlling the pH of an aqueous metal salt solution with the use of ammonia etc. to precipitate a hydroxide out of the aqueous metal salt solution, and then, drying or baking the precipitated hydroxide. The thus-obtained metal oxide may be subjected to pulverization or forming. For example, alumina can be generally prepared by forming a precipitate from an aqueous aluminum salt solution on the addition of ammonia etc. and forming and drying the precipitate. As the fluorination catalyst of the reaction step in the present invention, there can also be used γ-alumina commercially available for use as a catalyst support or a drying agent. Titania, zirconia and the like can be prepared in the same manner as above. Commercially available titania, zirconia and the like can also be used. Further, the metal oxide may be provided by coprecipitation in the form of a composite oxide and used as the fluorination catalyst of the reaction step in the present invention

As the fluorination catalyst, there can suitably be used a supported metal catalyst. The kind and amount of metal supported in the supported metal catalyst or the method of supporting the metal can be selected as appropriate based on the general knowledge of those skilled in the field of catalysts.

Examples of the supported metal catalyst are those each having a nitrate, a chloride, an oxide, a sulfate, a fluoride, a fluorochloride, an oxyfluoride, an oxychloride or an oxyfluorochloride of one or more kinds of metals selected from the group consisting of chromium, titanium, aluminum, manganese, nickel, cobalt, zirconium, iron, copper, silver, molybdenum and antimony supported on a support such as activated carbon or fluorinated alumina.

Examples of the activated carbon used as the support of the fluorination catalyst are: plant-based activated carbons prepared using wood, sawdust, wood charcoal, coconut shell charcoal, palm shell charcoal, raw ash etc. as raw materials; coal-based activated carbons prepared using peat coal, lignite, brown coal, bituminous coal, anthracite etc. as raw materials; petroleum-based activated carbons prepared using petroleum pitch, oil carbon etc. as raw materials; and activated carbons prepared using synthetic resins as raw materials. These activated carbons are commercially available and can be selected for use. For example, there can be used bituminous coal activated carbons (available under the trade name of Calgon granular activated carbon CAL from Calgon Carbon Japan K. K.) and coconut shell activated carbons (available by Japan EnviroChemicals Ltd.). The activated carbon is not however limited to these kinds of and these manufacturer's products. In general, the activated carbon is used in the form of particles. There is no particular limitation on the particle shape and size of the activated carbon. Further, the activated carbon can be used as it is or can be used after modified with hydrogen fluoride, hydrogen chloride, chlorofluorohydrocarbon or the like.

The amount of the metal supported on the support is generally in the range of 0.1 to 80 mass(capacity)%, preferably 1 to 50 mass%. If the amount of the metal supported is less than 0.1 mass%, the catalytic effect of the supported metal catalyst is small. On the other hand, it is difficult and unnecessary to support the metal in an amount of more than 80 mass% on the support.

As the method for preparation of the supported metal catalyst, it is feasible to dissolve a soluble compound of the above-mentioned at least one kind of metal in a solvent, and then, impregnate the support with the resulting solution or spray and adhere the resulting solution onto the support.

The solvent-soluble metal compound can be either a nitrate, a chloride, an oxide or a sulfate of the above metal. Specific examples of the solvent-soluble metal compound are chromium nitrate, chromium trichloride, chromium trioxide, potassium dichromate, iron chloride, iron sulfate, iron nitrate, titanium trichloride, titanium tetrachloride, manganese nitrate, manganese chloride, manganese dioxide, nickel nitrate, nickel chloride, cobalt nitrate, cobalt chloride, copper nitrate, copper sulfate, copper chloride, silver nitrate, copper chromite, copper dichromate, silver dichromate and sodium dichromate.

There is no particular limitation on the solvent as long as it is capable of dissolving the metal oxide and is not decomposed by reaction. Examples of the solvent are: water; alcohols such as methanol, ethanol and isopropanol; ketones such as methyl ethyl ketone and acetone; carboxylates such as ethyl acetate and butyl acetate; halogen compounds such as methylene chloride, chloroform and trichloroethylene; and aromatic compounds such as benzene and toluene. When the metal oxide is less soluble in water, the dissolution of the metal oxide in water can be promoted by the addition of an acid such as hydrochloric acid, nitric acid, sulfuric acid or nitrohydrochloric acid or an alkali such as sodium hydroxide, potassium hydroxide or aqueous ammonia as a dissolution aid.

Preferably, the fluorination catalyst is a nitrite, a chloride, an oxide or a sulfate of chromium, iron or copper supported on activated carbon in order to increase the reaction ate and the selectivity and yield of the trans-1,3,3,3-tetrafluoropropene. It is particularly preferable that the fluorination catalyst is in the form of an oxide, a fluoride, a chloride, a fluorochloride, an oxyfluoride, an oxychloride or an oxyfluorochloride of chromium supported on activated carbon.

In order to increase the reaction rate and the selectivity and yield of trans-1,3,3,3-tetrafluoropropene, the fluorination catalyst is also preferably a nitrate, a chloride, an oxide or a sulfate of chromium, iron or copper supported on fluorinated alumina.

Regardless of whether the fluorination catalyst is prepared by any method, it is effective to heat the fluorination catalyst together with a fluorinating agent such as hydrogen fluoride, fluorinated hydrocarbon or chlorinated hydrocarbon before use in the reaction in order to prevent a composition change in the fluorination catalyst during the reaction.

It is also effective to supply oxygen, chlorine, fluorinated or chlorinated hydrocarbon or the like into the reactor during the reaction in order to increase the life of the fluorination catalyst, the reaction rate and the yield of the trans-1,3,3,3-tetrafluoropropene.

The amount of the fluorination catalyst used in the reaction step is preferably 100 mass% or less based on the total amount of the raw material compounds supplied into the reactor. The amount of the target product compound may be unfavorably decreased if the amount of the fluorination catalyst used exceeds 100 mass%.

It suffices in the reaction step to supply the hydrogen fluoride in a stoichiometric amount or more relative to the 1-chloro-3,3,3-trifluoropropene to the reaction zone of the reactor. The mole ratio of the 1-chloro-3,3,3-trifluoropropene and hydrogen fluoride supplied to the reaction zone of the reactor is preferably in the range of 1-chloro-3,3,3-trifluoropropene:hydrogen fluoride = 1:8 to 1:25. If the supply amount of the hydrogen fluoride exceeds 25 mole times the supply amount of the 1-chloro-3,3,3-trifluoropropene, there may arises a problem in separating the unreacted hydrogen fluoride and the organic compounds such as target 1,3,3,3-tetrafuoropropene in the product A of the reaction step. On the other hand, the selectivity of the trans-1,3,3,3-tetrafuoropropene may be unfavorably decreased if the supply amount of the hydrogen chloride is less than 8 mole times the supply amount of the 1-chloro-3,3,3-trifluoropropene.

In the reaction step, the reaction temperature is preferably 200 to 450°C, more preferably 350 to 400°C. If the reaction temperature is lower than 200°C, the reaction is too slow to be practical. If the reaction temperature exceeds 450°C, the life of the fluorination catalyst becomes shortened. Further, the reaction proceeds quickly but generates a decomposition product, a macromolecular compound etc. so as to cause a deterioration in the selectivity of the trans-1,3,3,3-tetrafluoropropene unfavorably. In the reaction step, the equilibrium inside the reactor is shifted to the target compound side as the reaction temperature is increased. The reaction proceeds quickly as the reaction temperature becomes high. It is however practically desirable to avoid setting the reaction temperature to be higher than 450°C and more desirable to avoid setting the reaction temperature to be higher than 400°C in view of the deterioration of the catalyst under the high-temperature conditions, the limitation on the material of the reactor and the heat energy consumption and the like.

The reaction pressure is preferably equal to or lower than atmospheric pressure (barometric pressure) in the reaction step. However, the reactions pressure is not limited to the above and may be set higher than atmospheric pressure as long as the progress of the reaction is not inhibited under such pressure conditions that do not cause liquefaction of the hydrogen fluoride and the organic compounds in the reaction system of the reaction step. The reaction pressure is particularly preferably in the range of 0.01 to 0.3 MPa.

Further, the contact time (reaction step) is generally in the range of 0.1 to 300 seconds, preferably 3 to 60 seconds, in the reaction step. If the contact time is less than 0.1 second, there arises a possibility that the reaction may not proceed. The contact time is thus preferably set to 3 seconds or more. If the contact time exceeds 300 seconds, the cycle time may be too long. The contact time is thus preferably set to 60 seconds or less.

The optimal reaction conditions such as pressure and temperature for increasing the selectivity and yield of the trans-1,3,3,3-tetrafluoropropene vary depending on the catalyst used in the reaction step. It is preferable, in the case of using chromium chloride supported on fluorinated alumina or either an oxide, fluoride, chloride, fluorochloride, oxyfluoride, oxychloride or oxyfluorochloride of chlormium supported on activated carbon as the fluorination catalyst, to perform the reaction under the conditions of a pressure of 0.05 to 0.3 MPa and a temperature of 350 to 400°C. The selectivity and yield of the trans-1,3,3,3-tetrafluoropropene may be decreased if the reaction conditions are out of the above range.

In order to attain the high selectivity and yield of the trans-1,3,3,3-tetrafluoropropene relative to all of the organic compounds in the reaction product A, it is particularly preferable to perform the reaction in the gas phase under the conditions of a pressure of 0.05 to 0.3 MPa and a temperature of 350 to 400°C with the supply of the 1-chloro-3,3,3-trifluoropropene and hydrogen fluoride at a mole ratio of 1-chloro-3,3,3-trifluoropropene:hydrogen fluoride = 1:8 to 1:25 by the use of chromium chloride supported on fluorinated alumina or either an oxide, fluoride, chloride, fluorochloride, oxyfluoride, oxychloride or oxyfluorochloride of chlormium supported on activated carbon as the fluorination catalyst.

There is no particular limitation on the material of the reactor used in the reaction step as long as the reactor material has resistance to heat and to corrosion by hydrogen fluoride, hydrogen chloride etc. There can preferably be used a reactor formed of stainless steel, Hastelloy, Monel, Inconel, platinum or the like or a reactor formed with a lining of any of these metals.

In order to protect a surface of the catalyst from caulking, it is feasible in the reaction step to supply entrained gas such as oxygen, air or chlorine into the reaction zone or allow an inert gas such as nitrogen, argon or helium to coexist in the reaction zone. The supply amount of the gas is generally less than one time the total amount of the organic compound and hydrogen chloride supplied as the reactants. In the reaction step, the coexistence of the inert gas corresponds to reduced pressure conditions. If the supply amount of the gas is more than or equal to one time the total supply amount of the reactants, there may arises problems that: it becomes difficult to recover the reaction product in the subsequent step: there is a need for very large equipment to recover the reaction product in the subsequent step; and the productivity of the target compound is lowered.

It is feasible to activate the catalyst by any ordinary fluorination catalyst regeneration technique. For example, the fluorination catalyst can be regenerated i.e. activated by bringing the deteriorated catalyst into contact with dry air, chlorine, hydrogen fluoride etc. as appropriate at a temperature higher than or equal to the reaction temperature while controlling the generation of heat from the catalyst.

### 2.2 Rough Separation Step (Second Step)

Next, the rough separation step (second step) will be explained below.

The rough separation step is a step of distilling the reaction product A by a distillation column (occasionally referred to as "rough separation column"), which is located immediately after the reactor of the preceding step (reaction step), to separate and recover the unreacted 1-chloro-3,3,3-trifluoropropene and a major portion of the unreacted hydrogen fluoride as a distillation bottom product from the reaction product A and return the recovered 1-chloro-3,3,3-trifluoropropene and hydrogen fluoride to the reaction system of the preceding step (reaction step). Namely, the same distillation operation as that of liquid-phase reaction method is performed by direct coupling of the rough separation column to the gas-phase reactor in the rough separation step. This rough separation step is effective in the case where the unreacted raw organic compound and hydrogen fluoride are contained excessively in the reaction product. The residue B of the rough separation step contains not only an unrecovered remaining portion of the unreacted hydrogen fluoride but also the target trans-1,3,3,3-tetfaluoropropene and the by-produced chloride and other organic compounds. Although the composition of the residue B varies depending on the reaction method and conditions, the residue B generally contains 0.5 to 1 mol of the 1-chloro-3,3,3-trifluoropropene, 0.1 to 0.2 mol of the 1,1,1,3,3-pentafluoropropane, 1 to 1.5 mol of the hydrogen chloride and 0.5 to 10 mol of the hydrogen fluoride relative to 1 mol of the 1,3,3,3-tetrafluoropropene.

It is possible to easily supply an excessive amount of hydrogen fluoride into the reaction system of the reaction step and increase the selectivity and yield of the trans-1,3,3,3-tetrafluoropropene as mentioned above by recovering the unreacted 1-chloro-3,3,3-trifluoropropene and hydrogen fluoride in the rough separation step and returning these recovered unreacted reactants to the reaction step.

When the hydrogen fluoride is supplied excessively into the reaction system of the reaction step, there remains an excessive amount of unreacted hydrogen fluoride in the reaction product A. If such a reaction product A is subjected to distillation to extract the trans-1,3,3,3-tetrafluoropropene in the purification step, the load of the purification is increased. In the present invention, however, the major portion of the unreacted hydrogen fluoride is separated and recovered in the rough separation step; the residue B is supplied to the subsequent hydrogen fluoride separation step to separate and recover the remaining portion of the unreacted hydrogen fluoride; and the by-produced hydrogen chloride is separated in the hydrogen chloride separation step subsequent to the hydrogen fluoride separation step. It is thus possible to easily separate and recover the remaining portion of the unreacted hydrogen fluoride in the subsequent hydrogen fluoride separation step and reduce the loads of the hydrogen chloride separation step, dehydration drying step and purification step. It is also possible to prevent the hydrogen fluoride and hydrogen chloride from being mixed into the trans-1,3,3,3-tetrafluoropropene in the purification step so that the trans-1,3,3,3-tetrafluoropropene can be easily obtained with high purity.

The rough separation step, in which the unreacted 1-chloro-3,3,3-trifluoropropene and the major portion of the unreacted hydrogen fluoride are recovered by rough distillation from the reaction product A and returned to the reactor of the reaction step, is therefore essential in the production process of the present invention and important for efficient plant operation for production of trans-1,3,3,3-tetrafluoropropene.

As the distillation conditions in the rough separation step, the operation pressure is preferably set to 0.1 to 1.0 MPa. In the case where the operation pressure is set to atmospheric pressure (0.1 MPa), the temperature conditions are preferably set to a bottom temperature of 5 to 25°C and a top temperature of -20 to 5°C.

In the rough separation column, there can be used a packing material resistant to corrosion by hydrogen fluoride and hydrogen chloride. Examples of the packing material are: structured packing materials formed of metal such as stainless steel, nickel, Hastelloy or Monel or fluorocarbon resin such as tetrafluoroethylene resin, chlorotrifluoroethylene resin, vinylidene fluoride resin or tetrafluoroethylene-perfluoroalkyl vinylether copolymer (abbreviated as "PFA"); and random packing materials such as Lessing rings, Pall rings or Sulzer packings.

The number of stages of the rough separation column varies depending on the operation pressure and can be generally set to 15 or more under atmospheric pressure conditions.

Further, it is feasible to decrease a cooling heat-transfer surface of the rough separation column in the case where the rough separation step is performed under pressurized conditions. In the case where the rough separation step is performed under pressurized conditions, the rough separation column preferably has an inlet equipped with a compressor and an outlet equipped with a pressure regulating valve.

### 2.3 Hydrogen Fluoride Separation Step (Third Step)

The hydrogen fluoride separation step (third step) will be next explained below.

The hydrogen fluoride separation step is a step of, when the residue B containing the hydrogen fluoride, hydrogen fluoride, trans-1,3,3,3-tetrafluoropropene and other organic compounds is extracted from the rough separation column in the preceding rough separation step, removing the hydrogen fluoride from the distillated residue B.

For example, the hydrogen fluoride can be absorbed into sulfuric acid by contact of the residue B with sulfuric acid. More specifically, the hydrogen fluoride can be recovered by, upon contact of the residue B with the sulfuric acid, dividing into a liquid phase predominantly containing the hydrogen fluoride and sulfuric acid and a gas phase predominantly containing the organic compounds such as 1,3,3,3-tetrafluoropropene, 1-chloro-3,3,3-trifluoropropene and 1,1,1,3,3-pentafluoropropane and hydrogen chloride, and then, separating the hydrogen fluoride mainly from the liquid phase.

For the recovery of the hydrogen fluoride, the mass ratio of the sulfuric acid and hydrogen fluoride is generally in the range of sulfuric acid:hydrogen fluoride = 2:1 to 20:1, preferably 2:1 to 15:1, more preferably 2:1 to 10:1. If the proportion of the sulfuric acid is so small in the mixed system of the sulfuric acid and hydrogen chloride that the hydrogen fluoride is not dissolved sufficiently in the liquid phase and thus is entrained in the gas phase, it is unfavorably difficult to remove the hydrogen chloride due to the increase in the amount of hydrogen fluoride in the hydrogen chloride.

Although there can be used any apparatus and operation method for absorption of the hydrogen fluoride into the sulfuric acid in the hydrogen fluoride separation step, it is preferable to bring the residue B in a gas state into contact with the sulfuric acid. The liquid temperature of the sulfuric acid is thus preferably 10 to 50°C, more preferably 10 to 30°C, at atmospheric pressure (barometric pressure, 101325 Pa, the same applies to following). The sulfuric acid reacts with the hydrogen fluoride to form fluorosulfuric acid (fluosulfonic acid). The reaction operation may become unfavorably difficult if the temperature of the sulfuric acid is lower than 10°C. If the temperature of the sulfuric acid is higher than 50°C, the recovery rate may become unfavorably lowered due to polymerization of the 1,3,3,3-tetrafluoropropene and the like in the reaction product. The absorption of the hydrogen fluoride into the sulfuric acid can be done by blowing the reaction product gas into a tank filled with the sulfuric acid, or by blowing the reaction product gas into a packed washing tower to washing the gas with the sulfuric acid by counterflow contact. The absorption method is not however limited to the above. There can also be used any other absorption method.

It is feasible in the hydrogen fluoride separation step to separate and recover the hydrogen fluoride by heating the liquid phase, which predominantly contains the hydrogen fluoride and sulfuric acid, to thereby gasify the hydrogen fluoride, and then, condensing the gasified hydrogen fluoride in. The recovered hydrogen fluoride can be supplied again to the gas-phase reactor of the first reaction step.

### 2.4 Hydrogen Chloride Separation Step (Fourth Step)

The hydrogen chloride separation step (fourth step) will be next explained below.

The hydrogen chloride separation step is a step of removing the hydrogen chloride by water washing from the residue C remaining after the preceding hydrogen fluoride separation step.

For example, the hydrogen chloride can be removed from the residue C by subjecting the residue C to water washing and, more specifically, by bubbling the residue C into a water bath, or blowing the residue C into a packed washing tower for counterflow contact of the residue C with water, to absorb the hydrogen chloride into water and provide a liquid phase, i.e., hydrochloric acid, and then, separating the hydrochloric acid from the organic compounds such as 1,3,3,3-tetrafluoropropene, 1-chloro-3,3,3-trifluoropropene and 1,1,1,3,3-pentafluoropropane.

There can be used any apparatus and operation method for separation of the hydrogen chloride by water washing of the residue C in the hydrogen chloride separation step. The organic compounds separated from the hydrogen chloride can be recovered in a gas state or a liquid state. In the case where the content of the low-boiling trans-1,3,3,3-tetrafluoropropene (boiling point: -19°C) is high, it is preferable to absorb the hydrogen chloride into water by contact of the residue C in a gas state into contact with water, and then, separate the hydrogen chloride from the mixture of the organic compounds.

For the removal of the hydrogen chloride by water washing, the mass ratio of the water and hydrogen chloride is preferably in the range of water:hydrogen chloride = 3:1 to 20:1, more preferably 5:1 to 10:1, at room temperature (about 20°C, the same applies to following) and atmospheric pressure. At room temperature and atmospheric pressure, the solubility of hydrogen chloride in water is 25 mass% in a normal state and 37 mass% in a saturated state. If the solubility is higher than this level, there unfavorably occurs evaporation and vaporization of excessive hydrogen chloride.

Upon recovery of the hydrochloric acid by absorption of the hydrogen chloride into water in the hydrogen chloride separation step, it is feasible to purify the recovered hydrochloric acid by any known technique, e.g., by adsorption of impurities such as hydrogen fluoride and organic compounds onto an adsorbent such as zeolite.

Alternatively, the hydrogen fluoride can be recovered by, in place of water washing, contact of the residue C of the preceding hydrogen fluoride separation step with an aqueous sodium hydroxide solution.

### 2.5 Dehydration Drying Step (Fifth Step)

Next, the dehydration drying step (fifth step) will be explained below.

The dehydration drying step is a step of dehydrating and drying the residue D remaining after the preceding hydrogen chloride separation step.

The residue D contains at least water due to the water washing or contact with the aqueous sodium hydroxide solution in the preceding hydrogen chloride separation step. The residue D also contains entrained water mist. This water-containing reaction product can be dehydrated and dried by freezing and solidifying the water by means of a heat exchanger or by adsorbing the water onto an adsorbent such as zeolite in the dehydration drying step.

The dehydration method using the adsorbent, e.g., dehydration by contact with a specific zeolite is superior as it is practicable regardless whether the 1,3,3,3-tetrafluoropropene is in a gas state or a liquid state. Due to the fact that the residue D of the preceding hydrogen chloride separation step is given as a mixed gas containing water vapor at a water content of 1000 ppm or more and is generally 230 times larger in volume than as a liquid, it is necessary, in the case where this dehydration method is carried out with the use of a zeolite-packed dehydration column, to increase the mass flow rate of the water-containing mixed gas through the dehydration column per unit time to a level appropriate for industrial production. However, the capacity of the dehydration column needs to be increased with the mass flow rate of the mixed gas through the dehydration column. This leads to the problems that: the zeolite has to be used in a large amount as the dehydration agent and has to be regenerated.

By contrast, the dehydration method using the heat exchanger enables freezing and removing of the water contained in the residue D simultaneously with condensation of the 1,3,3,3-tetrafluoropropene and thus has the advantage over the conventional dehydration method using the zeolite-packed dehydration column in that it allows not only size reduction and simplification of dehydration equipment but also easy dehydration operation.

Even when water is contained in an excessive amount larger than or equal to its saturation content in the residue D after the preceding hydrogen chloride separation step, i.e. the mixed gas containing the organic compounds such as 1,3,3,3-tetrafluoropropene, 1-chloro-3,3,3-trifluoropropene and 1,1,1,3,3-pentafluoropropane, it is possible to dehydrate the mixed gas to almost no water content by adopting the above dehydration method for freezing and removing the water by the heat exchanger in the dehydration drying step, i.e., introducing the mixed gas into the heat exchanger while controlling the setting temperature of the heat exchanger to be lower than the condensation temperatures of these organic compounds and thereby cooling and condensing the mixed gas.

There can suitably be used, as the heat exchanger for freezing and removing the water from the mixed gas in the dehydration drying step, an indirect heat exchanger that allows heat exchange between the residue D and cooling medium via a cooling heat-transfer surface. Examples of the indirect heat exchanger are those of double-tube type, cylindrical multi-tube type, cylindrical coil type and cylindrical jacketed type. An external jacket may be attached to the cylindrical multi-tube heat exchanger or cylindrical coil heat exchanger for increase of heat transfer surface area.

Preferably, the heat exchanger is formed of a metal material of high thermal conductivity. Examples of the metal material of the heat exchanger are iron, iron steel, copper, lead, zinc, brass, stainless steel, titanium, aluminum, magnesium, Monel, Inconel and Hastelloy. It is further preferable to apply a lining of resin, ceramic or glass to the cooling heat-transfer surface of the heat exchanger in the case where any corrosive substance is contained in the residue D.

Although the heat transfer surface area of the heat exchanger depends on the temperature of the cooling medium, it is preferable that the heat transfer surface area of the heat exchanger is enough to exchange a sufficient amount of heat required for condensation of the gaseous residue D and for freezing of the water contained in the residue D. In view of the fact that the heat transfer coefficient of the heat exchanger becomes decreased due to adhesion of the water to the cooling heat-transfer surface of the heat exchanger, the heat transfer surface area of the heat exchanger is preferably at least 1.5 times or larger than its theoretically required value.

Further, a fin may be attached to the heat-transfer surface of the heat exchanger. It is particularly effective for improvement in heat transfer efficiency to attach the fin to the side of the heat-transfer surface with which the residue D comes into contact and thereby increase the heat transfer surface area of the heat exchange.

For the introduction of the residue D into the heat exchanger, there can be adopted a flow system that allows the residue D to flow through the heat exchanger of sufficient heat transfer surface area. In the case where the heat exchanger is of vertical type, the residue D, i.e., mixed gas is preferably introduced from the top side of the heat exchanger. In this case, the freezing of the water occurs to cause a blockage from the top side of the cooling heat-transfer surface of the heat exchanger. It is thus desirable to provide a plurality of introduction holes in the bottom side of the heat exchanger and change the position of introduction of the residue D to the bottom side. Even in the case where the heat exchanger is of horizontal type, the residue D is also preferably introduced from the top side of the heat exchanger. A plurality of introduction holes may be provided in a line.

There is no particular limitation on the setting temperature of the cooling heat-transfer surface of the heat exchanger, i.e. cooling temperature. The dehydration operation needs to be performed at a cooled temperature at which the gaseous trans-1,3,3,3-tetrafluoropropene (boiling point: -19°C) gets condensed under operation pressure conditions. The cooling temperature is generally -50 to -20°C, preferably -40 to -25°C, under atmospheric pressure conditions. If the temperature is higher than -20°C, it is difficult to condense the 1,3,3,3-tetrafluoropropene. In this operation, a receiving tank is disposed on the bottom side of the heat exchanger so as to, when the mixed gas is liquefied by condensation for freezing and removal of the water, receive and recover the liquefied mixed in the receiving tank. The temperature of the receiving tank is preferably lower than or equal to the condensation temperature of the trans-1,3,3,3-tetrafluoropropene. It is feasible to dispose a U-shaped or coil-shaped tube in the receiving tank for further freezing and removal of the water in the residue D containing the liquefied 1,3,3,3-tetrafluoropropene, 1-chloro-3,3,3-trifluoropropene, 1,1,1,3,3-pentafloropropane and the like.

There is no particular limitation on the cooling medium used in the heat exchanger. The cooling medium can be selected from an aqueous medium, an inorganic brine and an organic brine depending on the cooling temperature.

The heat of vaporization of the liquefied trans-1,3,3,3-tetrafluoropropene may be used as cooling means. In the case of using the condensation of a gas by a heat exchanger as an intermediate purification technique in an industrial production method, it is conceivable to the vaporize the liquefied gas and then feed the vaporized gas to a distillation column of the subsequent purification step. The load of heating and heat removal of the external heating source can be removed when the liquefied gas is vaporized on the cooling-medium-flow side of the heat exchanger dehydration equipment. This method is also effective in terms of energy conservation and is thus suitably adopted in the production process of the present invention.

It is feasible to dehydrate the residue D by the heat exchanger under pressurized conditions. The pressure of the mixed gas inside the heat exchanger is generally preferably 0.1 to 1 MPa. The cooling temperature under such pressurized conditions can be set as appropriate depending on the operation pressure.

In the flow system, the linear velocity of the residue D to be dehydrated in the heat exchanger is 30 to 1200 m/hr, preferably 60 to 600 m/hr. If the linear velocity is lower than 30 m/hr, the dehydration operation time may become unfavorably long. If the linear velocity is higher than 1200 m/hr, the freezing of the water and the condensation of the organic compounds in the residue D may become unfavorably insufficient.

Further, the amount of the frozen water adhered to the cooling heat-transfer surface of the heat exchanger dehydration equipment increases with the time of contact of the water-containing mixed gas in the flow system. It is thus necessary to melt and remove the frozen water after the lapse of a predetermined time period. As the means for melting and removing the frozen water, there can be used a technique of flowing a dried inert gas of 5 to 200°C through the dehydration equipment from the top side. The temperature of the inert gas may be set to a high temperature. It is however desirable that the temperature of the inert gas is 20 to 100°C for less thermal stress load on equipment material and for energy conservation in the heat exchanger dehydration equipment. As the heating means for melting the frozen water, there can be used a technique of allowing a heating medium to flow through the portion of the heat exchanger opposed to the portion through which the cooling medium flows. At this time, the cooling medium and the heating medium are not limited to be the same material or different materials. There is no particular limitation on the above-mentioned inert gas. In terms of cost efficiency, dry air or dry nitrogen is preferably used as the inert gas.

The melted frozen water can be discharged from the bottom side of the heat exchanger dehydration equipment in the form of water or water vapor. This organic-containing melted water can be used as a wash water in the precedent hydrogen chloride separation step.

Furthermore, it is preferable to perform a water separation step using a mist separator etc. between the hydrogen chloride separation step and the dehydration drying step. The residue D contains at least water due to the water washing or contact with the aqueous sodium hydroxide solution in the preceding hydrogen chloride separation step and also contains entrained water mist as mentioned above. The total water content of the residue D after the water washing operation of the hydrogen chloride separation step is 3000 ppm to 10 %. When the water separation step is performed with the use of the mist separator etc. between the hydrogen chloride separation step and the dehydration drying step, the water content of the residue D can be lowered to the order of 1300 ppm. It is possible to lower the water content of the residue E to be smaller than 100 ppm by freezing and removing the water in the residue D by means of the heat exchanger in the dehydration drying step after dehydrating the residue D by means of the mist separator.

In the case where further reduction in the water content of the condensed liquid of the residue D is desired, it is feasible to dry the residue D by contact with a dehydration agent such as calcium chloride, calcium oxide, magnesium sulfate or phosphorus pentaoxide or an adsorbent such as silica gel or zeolite after the dehydration drying step.

### 2.6 Purification Step (Sixth Step)

The purification step (sixth step) will be explained below.

The purification step is a step of purifying the trans-1,3,3,3-tetrafluoropropene, i.e., extracting the trans-1,3,3,3-tetrafluoropropene by distillation of the residue E remaining after the preceding dehydration drying step.

The distillation operation can be carried out in a batch system or a continuous system. Although the operation pressure can be set to any pressure such as atmospheric pressure (barometric pressure) or pressurized conditions, it is preferable to set the operation pressure conditions capable of increasing the condensation temperature in the distillation.

The purification step will be explained below in more detail by taking, as one example, the case where the distillation operation is performed through the use of a pair of first and second distillation columns. However, the distillation can alternatively be carried out by more than two distillation columns or by a batch system.

First, the residue E is subjected to distillation by the first distillation column so as to extract and recover the trace amounts of low-boiling by-products such as 3,3,3-trifluoropropyne and 2,3,3,3-tetrafluoropropene contained in the residue E from the top of the first distillation column. The top distillate of the first distillation column is returned to the reaction system of the reaction step, that is, supplied into the gas-phase reactor and reused in the reaction step. On the other hand, the distillation bottom product of the first distillation column is subjected to distillation by the second distillation column so as to extract and recover the target trans-1,3,3,3-tetrafluoropropene from the top of the second distillation column and, at the same time, recover the low-boiling distillation bottom product containing cis-1,3,3,3-tetrafluoropropene, 1-chloro-3,3,3-trifluoropropene and 1,1,1,3,3-pentafluoropropane. The recovered distillation bottom product is supplied into the reaction system of the first reaction step and reused as the raw material. It is feasible to separate and purify by e.g. extractive distillation the mixture of cis-1,3,3,3-tetrafluoropropene, 1-chloro-3,3,3-trifluoropropene and 1,1,1,3,3-pentafluoropropane recovered as the distillation bottom product of the second distillation column.

There is no particular limitation on the distillation column used in the purification step as long as the distillation column has a wall surface inert to the distillate. The distillation column may be of the type having a wall surface formed of glass or stainless steel or of the type having a lining of tetrafluoroethylene resin, chlorotrifluoroethylene resin, vinylidene fluoride resin, PFA resin or glass on a substrate of steel etc. Further, the distillation column may be in the form of a trayed column or a packed column packed with a packing material such Raschig rings, Lessing rings, Dixon rings, Pall ring, Intalox saddles or Sulzer packings

Although the distillation operation can be carried out at atmospheric pressure, it is preferable to carry out the distillation operation under pressurized conditions in order to decrease the pressure loss of the distillation column and reduce the load of the condenser. There is no particular limitation on the number of stages of the distillation column. The number of stages of the distillation column is preferably 5 to 100, more preferably 10 to 50. If the number of stages of the distillation column is less than 5, the purity of the trans-1,3,3,3-tetrafluoropropene may not be improved to a sufficiently high level. If the number of stages of the distillation column exceeds 100, there unfavorably occur increases in the economical load of the distillation column itself and in the time required for the distillation operation.

### 2.7 Other Steps

In the present invention, the following step may preferably additionally be performed after the purification step.

When the trans-1,3,3,3-ttrafluoropropene is extracted by distillation of the residue E (after the preceding dehydration drying step) in the purification step, the resulting distillation residue F containing the 1-chloro-3,3,3-trifluoropropane and cis-1,3,3,3-tetrafluoropropene can be supplied to the reaction step. It is possible by this step to secure further resupply of the unreacted reactant in addition to the effective use of the by-product.

It is possible to obtain the trans-1,3,3,3-tetrafluoropropene more efficiently by supplying the distillation residue F to the reaction system after converting the cis-1,3,3,3-tetrafluoropropene in the distillation residue F to 1,1,1,3,3-pentafluoropropane.

The cis-1,3,3,3-tetrafluoropropene is preferably converted to 1,1,1,3,3-pentafluoropropane by reaction with excessive hydrogen fluoride in a gas phase in the presence of a solid catalyst having antimony pentachloride, antimony trichloride, antimony pentabromide, antimony tribromide, tin tetrachloride, titanium tetrachloride, molybdenum pentachloride, tantalum pentachloride, niobium pentachloride or the like supported on a catalyst support such as activated carbon, fluorinated alumina or fluorinated zirconia or by reaction with hydrogen fluoride in a liquid phase in the presence of a catalyst such as antimony pentachloride, antimony trichloride, antimony pentabromide, antimony tribromide, tin tetrachloride, titanium tetrachloride, molybdenum pentachloride, tantalum pentachloride or niobium pentachloride. It is particularly preferable to convert the cis-1,3,3,3-tetrafluoropropene to 1,1,1,3,3-pentafluoropropane by continuous reaction with hydrogen fluoride in the presence of a catalyst having antimony pentachloride supported on activated carbon. There are disclosed techniques of conversion of 1-chloro-3,3,3-trifluoropropane or cis-1,3,3,3-tetrafluoropropene to 1,1,1,3,3-pentafluoropropane in Patent Documents 8 to 13. Any of these known techniques can be adopted in this step. Further, the excessive hydrogen fluoride discharged out of the reactor can be returned as it is, together with the generated 1,1,1,3,3-pentafluoropropane, to the reaction system of the reaction step.
The distillation residue F may be used, after conversion to 1,1,1,3,3-pentafluoropropane, effectively for another purpose.

### 3. Example of Production Process of Trans-1,3,3,3-tetrafluoropropene

One example of the production Process of the trans-1,3,3,3-tetrafluoropropene according to the present invention will be described below with reference to FIG. 1. FIG. 1 is an example of a flow chart showing the production process of the trans-1,3,3,3-tetrafluoropropene according to the present invention. The present invention is not however limited to this flow chart.

First, trans-1,3,3,3-tetrafluoropropene is formed by reaction of 1-chloro-3,3,3-trifluoropropene and hydrogen fluoride as raw materials *a* in the presence of a fluorination catalyst in a gas-phase reactor 1 in the reaction step (first step).

Next, the resulting product A of the reaction step is supplied to and distilled by a rough separation column 2 in the rough separation step (second step). In the rough separation column 2, the reaction product A is separated into a residue B containing the trans-1,3,3,3-tetrafluoropropene, hydrogen chloride, hydrogen fluoride and other organic compounds and a distillation bottom product b containing the unreacted 1-chloro-3,3,3-trifluoropropene and hydrogen fluoride. The distillation bottom product b is supplied into the gas-phase reactor 1 and reused as the raw material.

Subsequently, the residue B is supplied to a hydrogen fluoride absorption column 3. In the hydrogen fluoride absorption column 3, the hydrogen fluoride in the residue B is absorbed into sulfuric acid upon contact of the residue B with the sulfuric acid in the hydrogen fluoride separation step (third step). The resulting mixture c containing the sulfuric acid and hydrogen fluoride is fed to a diffusion column 4 to recover hydrogen fluoride d from the mixture c. The recovered hydrogen fluoride d is supplied into the gas-phase reactor 1 and reused as the raw reactant material.

In the hydrogen chloride separation step (fourth step), the residue C remaining after the recovery of the hydrogen fluoride *d* is supplied to a hydrogen chloride absorption column 5. In the hydrogen chloride absorption column 5, the residue C is washed with water or an aqueous sodium hydroxide solution by e.g. bubbling to separate hydrogen chloride *e* from the residue C.

In the dehydration drying step (fifth step), the residue D remaining after the removal of the hydrogen chloride *e* is introduced into a mist separator 6 as needed. In the mist separator 6, water *h1* is removed from the residue D. The residue D is fed to and cooled by a heat exchanger 7, thereby removing water h2 from the residue D by freezing while condensing the residue D from a gas to a liquid.

The product E of the above dehydration operation is fed to and distilled by a rectification column 8 to purify the trans-1,3,3,3-tetrafluoropropene in the purification step (sixth step). The resulting distillation residue F may be supplied to the gas-phase reactor 1 and reused as the raw material.

The distillation residue F remaining after the purification of the trans-1,3,3,3-tetrafluoropropene, which contains the 1-chloro-3,3,3-trifluoropropene and cis-1,3,3,3-tetrafluoropropene, may be returned to the reaction step (first step) after converting the cis-1,3,3,3-tetrafluoropropene in the distillation residue F to 1,1,1,3,3-pentafluoropropane.

### Examples

The present invention will be described in more detail below by way of the following examples. It should be however noted that the present invention is not limited to the following examples. In the following examples, the composition of the reaction product A or the residue B were measured with a gas chromatograph (GC) using a hydrogen flame ionization detector (FID) by direct injection of the reaction product A or the residue B into the GC. The composition ratio of the respective components is given in mol% based on the peak areas of the GC chart.

### [Preparation of Fluorination Catalysts]

Fluorination catalysts for the formation reaction of trans-1,3,3,3-tetrafluoropropene were prepared by the following procedures.

### [Catalyst Preparation Example 1]

In this example, the fluorination catalyst was prepared by providing fluorinated alumina upon contact of activated alumina with hydrogen fluoride, and then, supporting chromium on the fluorinated alumina. The detailed catalyst preparation procedure is as follows.

First, 1200 g of activated alumina of 2 mm to 4 mm particle size (available from Sumitomo Chemical Co., Ltd. under the trade name of "NKHD-24", specific surface: 340 m²/g) was weighed out and washed. Further, 10 mass% hydrofluoric acid was prepared by dissolving 460 g of hydrogen fluoride into 4140 g of water. While stirring the 10 mass% hydrofluoric acid, the washed activated alumina was gradually added to the 10 mass% hydrofluoric acid. The resulting mixture was left still for 3 hours. After that, the activated carbon was washed with water, filtered out, and then, dried by heating at 200°C in an electric furnace for 2 hours. A gas-phase reaction apparatus was packed with 1600 ml (1600 cm³) of the dried activated alumina. Herein, the gas-phase reaction apparatus used was a gas-phase reactor having a cylindrical reaction tube of stainless steel (SUS316L) and an outer sleeve connected to a heating medium circulation device. While flowing nitrogen through the cylindrical reaction tube, the heating medium circulation device was operated to circulate a heating medium of 200°C and thereby heat the cylindrical reaction tube. Subsequently, hydrogen fluoride was introduced into the reaction tube together with nitrogen at a mass ratio of HF/N₂ = 1/10 to 1/5. As the temperature of the activated alumina was increased upon introduction of the hydrogen fluoride, the flow rates and ratio of the hydrogen fluoride and nitrogen were controlled in such a manner that the temperature of the activated alumina did not exceed 350°C. At the time of completion of heat generation, the setting temperature of the heating medium was changed to 450°C. The introduction of the hydrogen fluoride and nitrogen was continued for another 2 hours. With this, the fluorinated alumina was obtained. Next, 1000 ml (1000 cm³) of aqueous CrCl₃ solution was prepared by dissolving 2016 g of commercially available special grade reagent, CrCl₃.6H₂O, into pure water. In this aqueous solution, 1500 ml (1500 cm³) of the fluorinated alumina was immersed. The resulting mixture was left still for one day. The resulting fluorinated alumina was filtered out and dried by heating at 100°C in a hot-air circulation type drying device for one day. The above-obtained chromium-supporting fluorinated alumina was packed into a cylindrical reaction tube of SUS316L of 5 cm in diameter and 90 cm in length. While flowing nitrogen gas through the reaction tube, the reaction tube was heated to 300°C. At the time the distillation of water from the reaction tube was no longer seen, hydrogen fluoride was introduced into the reaction tube together with nitrogen gas. The concentration of the hydrogen fluoride was gradually increased. The temperature of the reaction tube was raised to 450°C when a hot spot, which was higher in temperature than its surroundings due to adsorption of the hydrogen fluoride onto the packed chromium-supporting fluorinated alumina, reached the outlet end of the reaction tube. The reaction tube was kept heated at 450°C for 1 hour. In this way, the fluorination catalyst was completed.

### [Catalyst Preparation Example 2]

In 150 g of pure water, 100 g of coconut shell pulverized activated carbons under 4x10 mesh size (available from Calgon Carbon Japan K. K. under the trade name of "PCB" was immersed. Further, a solution was separately prepared by dissolving 40 g of CrCl₃-6H₂O (special grade reagent) into 100 g of pure water. The above prepared activated carbon was mixed and stirred in the separately prepared solution. The resulting mixture was left still for one day. After that, the activated was filtered out and baked by heating at 200°C in an electric furnace for 2 days. The above-obtained chromium chloride-supporting activated carbon was packed into a cylindrical reaction tube of SUS316L of 5 cm in diameter and 90 cm in length. While flowing nitrogen gas through the reaction tube, the reaction tube was heated to 200°C. At the time the distillation of water from the reaction tube was no longer seen, hydrogen fluoride was introduced into the reaction tube together with nitrogen gas. The concentration of the hydrogen fluoride was gradually increased. The temperature of the reaction tube was raised to 450°C when a hot spot, which was higher in temperature than its surroundings due to adsorption of the hydrogen fluoride onto the packed chromium-supporting activated carbon, reached the outlet end of the reaction tube. The reaction tube was kept heated at 450°C for 1 hour. In this way, the fluorination catalyst was completed.

### [Formation of 1,3,3,3-Tetrafluoropropene (Reaction Step)]

The formation reaction of trans-1,3,3,3-tetrafluoropropene (trans-TFP) from 1-chloro-3,3,3-trifluoropropene (CTFP) and hydrogen fluoride (HF) was carried out in the gas-phase reactor 1 with the use of the fluorination catalyst obtained in either Catalyst Preparation Example 1 or 2. In the reaction, the flow rate of the 1-chloro-3,3,3-trifluoropropene (CTFP) was kept constant; the flow rate of the hydrogen fluoride (HF) was set to 0.25 g/min or 0.49 g/min; the reaction temperature was set to 360°C or 380°C; and the pressure inside the reactor was set to 0.1 MPa or 0.2 MPa. The detailed reaction procedure is as follows.

As the gas-phase reactor 1, a cylindrical reaction tube of stainless steel (SUS316L) of 1 inch (about 2.54 cm) in diameter and 30 cm in length was used. Into the cylindrical reaction tube of the gas-phase reactor 1, 50 ml (50 cm³) of the fluorination catalyst of Catalyst Preparation Example 1 or 2 was packed.

The reaction tube of the gas-phase reactor 1 was heated to 200°C while flowing nitrogen through the reaction tube at a flow rate of 30 ml/min (30 cm³/min). Subsequently, hydrogen fluoride was introduced into the reaction tube at a flow rate of 0.10 g/min. While flowing the hydrogen fluoride together with the nitrogen gas through the reaction tube, the reaction tube was kept heated at 450°C for 1 hour.

After that, the temperature of the reaction tube was lowered to 360°C or 380°C. Hydrogen fluoride (HF) was then supplied into the gas-phase reactor 1 at a flow rate of 0.25 g/min or 0.49 g/min. Simultaneously, vaporized 1-chloro-3,3,3-trifluoropropene (CTFP) was supplied into the gas-phase reactor 1 at a flow rate of 0.16 g/min.

The reaction was stabilized after a lapse of 1 hour from the initiation of the reaction. The product gas extracted from the gas-phase reactor 1 as the reaction product A was blown into water for 2 hours to remove therefrom an acid gas component. The product gas was then fed to a dry ice-acetone trap, thereby collecting 6.0 to 8.0 g of organic substance in the trap. The collected organic substance was analyzed by gas chromatography.

When the flow rate of the 1-chloro-3,3,3-trifluoropropene (CTFP) was 0.16 g/min and the flow rate of the hydrogen fluoride (HF) was 0.25 g/min, the supply mole ratio was CTFP:HF = 1:8. On the other hand, the supply mole ratio was CTFP:HF = 1:20 when the flow rate of the 1-chloro-3,3,3-trifluoropropene (CTFP) was 0.16 g/min and the flow rate of the hydrogen fluoride (HF) was 0.49 g/min.

The composition ratio of the reaction product A was measured by GC-FID. The measurement results of the composition ratio of the reaction product A (the selectivity of the respective product components) relative to the reaction conditions are indicated in TABLE 1. These measurement results were determined, in units of mol%, from the peak areas of the respective organic compounds of the GC-FID chromatogram chart according to the area percentage method assuming the total peak area of the chromatogram chart as 100%.

**TABLE 1**

| Catalyst used | CTFP (g/min) | HF (g/min) | Temp. (°C) | Pressure (MPa) | Product components (mol%) | | | |
|---|---|---|---|---|---|---|---|---|
| | | | | | Trans-TFP | Cis-TFP | PFP | CTFP |
| Preparation Example 1 | 0.16 | 0.25 | 360 | 0.1 | 32.1 | 7.6 | 11.5 | 48.8 |
| | 0.16 | 0.25 | 380 | 0.1 | 34.5 | 7.9 | 10.5 | 47.1 |
| | 0.16 | 0.25 | 360 | 0.2 | 28.3 | 5.8 | 28.5 | 37.4 |
| | 0.16 | 0.49 | 360 | 0.1 | 44.4 | 9.0 | 11.3 | 35.3 |
| | 0.16 | 0.49 | 380 | 0.1 | 46.6 | 9.5 | 8.0 | 35.9 |
| Preparation Example 2 | 0.16 | 0.25 | 360 | 0.1 | 30.3 | 6.2 | 15.8 | 47.7 |
| | 0.16 | 0.25 | 380 | 0.1 | 33.1 | 7.1 | 12.6 | 47.2 |

| | | | | | | | | |
|---|---|---|---|---|---|---|---|---|
| Amount of catalyst used: 50 ml Trans-TEP: trans-1,3,3,3-tetrafluoropropene Cis-TEP: cis-1,3,3,3-tetrafluoropropene PEP: 1,1,1,3,3-pentafluoropropane CTFP: 1-chloro-3,3,3-trifluoropropene Other product components: 3,3,3-trifluoropropyne, 2,3,3,3-tetrafluoropropene etc. | | | | | | | | |

In the case of using the catalyst of Catalyst Preparation Example 1, the selectivity of the trans-1,3,3,3-tetrafluoropropene (trans-TFP) was e.g. 32.1 mol% or 34 mol% when the HF flow rate was 0.25 g/min; and the selectivity of the trans-1,3,3,3-tetrafluoropropene (trans-TFP) was 44.4 mol% or 46.6 mol% when the HF flow rate was 0.49 g/min. The selectivity of the trans-1,3,3,3-tetrafluoropropene (trans-TFP) was thus higher when the HF flow rate was 0.49 g/min than when the HF flow rate was 0.25 g/min. When the other reaction conditions were the same, the selectivity of the trans-1,3,3,3-tetrafluoropropene (trans-TFP) was higher at a reaction temperature of 380°C than at a reaction temperature of 360°C. More specifically, the selectivity of the trans-1,3,3,3-tetrafluoropropene (trans-TFP) was 32.1 mol% at a reaction temperature of 360°C and 34.5 mol% at a reaction temperature of 380°C when the HF flow rate was 0.25 g/min, as shown in TABLE 1, in the case of using the catalyst of Catalyst Preparation Example 1. Further, the selectivity of the trans-1,3,3,3-tetrafluoropropene (trans-TFP) was 44.4 mol% at a reaction temperature of 360°C and 44.6 mol% at a reaction temperature of 380°C when the HF flow rate was 0.49 g/min.

In the case of using the catalyst of Catalyst Preparation Example 2, the selectivity of the trans-1,3,3,3-tetrafluoropropene (trans-TFP) was 30.3 mol% at a reaction temperature of 360°C and 33.1 mol% at a reaction temperature of 380°C when the HF flow rate was 0.25 g/min.

Moreover, the reaction was carried out with the use of the catalyst of Catalyst Preparation Example 1 by supplying hydrogen fluoride (HF) and vaporized 1-chloro-3,3,3-trifluoropropene (CTFP) into the gas-phase reactor 1 at a flow rate of 0.25 g/min or 0.49 g/min and a flow rate of 0.16 g/min, respectively, while setting the temperature of the reaction tube to 150°C. The reaction was stabilized after a lapse of 1 hour from the initiation of the reaction. The product gas extracted from the gas-phase reactor 1 as the product (B) was blown into water for 2 hours to remove therefrom an acid gas component. The product gas was then fed to a dry ice-acetone trap, thereby collecting 8.5 g of organic substance in the trap. The collected organic substance was analyzed by gas chromatography. Most of the collected organic substance was unreacted 1-chloro-3,3,3-trifluoropropene (CTFP). The selectivity of the target trans-1,3,3,3-tetrafluoropropene (trans-TFP) was lower than 1% and did not reach a satisfactory level. The reason for such reaction results is assumed to be that the reaction temperature was too low.

### [Formation and Recycling of 1,3,3,3-Tetrafluoropropene (Reaction Step + Rough Separation Step)]

### [Process Example 1]

As the gas-phase reactor 1, a cylindrical reaction tube of stainless steel (SUS316L) of 52.7 cm in inside diameter and 58 cm in length was provided. The gas-phase reactor 1 was packed with 1200 ml (1200 cm³) of the fluorination catalyst of Catalyst Preparation Example 1.

Further, a distillation column was provided as the rough separation column 2 at a downstream side of the gas-phase reactor 1. A cooling condenser was arranged at a top side of the distillation column to liquefy the top distillate whereas a heating bath was arranged at a bottom side of the distillation column to heat the distillation bottom product. The rough separation column 2 was 54.9 mm in inside diameter and 40 cm in length and was packed with 6 mm Raschig rings.

The formation reaction of trans-1,3,3,3-tetrafluoropropene (trans-TFP) from 1-chloro-3,3,3-trifluoropropene (CTFP) and hydrogen fluoride (HF) was carried out in the gas-phase reactor 1. In the reaction, the reaction temperature was set to 360°C; the reaction pressure was set to 0.2 MPa: and the flow rate of the hydrogen fluoride (HF) was set to 6.0 g/min. Further, the 1-chloro-3,3,3-trifluoropropene (CTFP) was vaporized in advance and supplied into the gas-phase reactor 1 at a flow rate of 3.8 g/min. The supply mole ratio was CTFP:HF = 1:10.

The formation reaction of the trans-1,3,3,3-tetrafluoropropene (trans-TFP) was stabilized after a lapse of 2 hours from the initiation of the reaction. After that, the resulting product gas was introduced as the reaction product A into the rough separation column 2.

The distillation conditions of the rough separation column 2 and the measurement results of the composition of the distillate (residue B) are indicated in TABLE 2.

**TABLE 2**

| | Heating bath temp. (°C) | Cooling condenser temp. (°C) | Pressure (MPa) | Distillation rate (mol%) | | | Conc. (mol%) of trans-TFP in organic substance | | Conc. ratio |
|---|---|---|---|---|---|---|---|---|---|
| | | | | Organic substance | HF | HCl | Inlet | Outlet | In./Out. |
| Conditions 1 | 24 | -5 | 0.2 | 48.1 | 6.9 | 91.8 | 26.0 | 55.6 | 2.1 |
| Conditions 2 | 25 | 1 | 0.2 | 63.8 | 10.2 | 91.5 | 26.0 | 42.5 | 1.6 |

As shown in TABLE 2, the distillation operation was performed under two kinds of distillation conditions by varying the setting temperature of the heating bath and the setting temperature of the cooling condenser. More specifically, the product gas was distillated by the rough separation column 2 under conditions 1 where the heating bath temperature was 24°C, the cooling condenser temperature was -5°C and the pressure inside the rough separation column 2 was 0.2 MPa and under conditions 2 where the heating bath temperature was 25°C, the cooling condenser temperature was 1°C and the pressure inside the rough separation column 2 was 0.2 MPa. The hydrogen fluoride (HF) and hydrogen chloride (HCl) was quantified by titration. In TABLE 2, the term "distillation rate" was defined in mol% as the amount of the product component i.e. organic substance, HF or HCl contained in the residue B after the rough distillation operation assuming the amount of the product component i.e. organic substance, HF or HCl contained in the reaction product A as 100 and was determined by dividing the molar amount of the product component at the outlet of the rough separation column 2 (the molar amount of the product component in the residue B) by the molar amount of the product component at the inlet of the rough separation column 2 (the molar amount of the product component in the reaction product A).

Under the conditions 1, the distillation rate of the organic substance, hydrogen fluoride (HF) and hydrogen chloride was 48.1 mol%, 6.9 mol% and 91.8 mol%, respectively; the concentration of the trans-1,3,3,3-tetrafluoropropene (trans-TFP) in the organic substance was 26.0 mol% at the inlet of the rough separation column and 55.6 mol% at the outlet of the rough separation column; and the concentration ratio was 2.1. Under the conditions 2, on the other hand, the distillation rate of the organic substance, hydrogen fluoride (HF) and hydrogen chloride was 63.8 mol%, 10.2 mol% and 91.5 mol%, respectively; the concentration of the trans-1,3,3,3-tetrafluoropropene (trans-TFP) in the organic substance was 26.6 mol% at the inlet of the rough separation column and 42.5 mol% at the outlet of the rough separation column; and the concentration ratio was 2.1.

The composition of the organic substance was also measured by GC when the distillation operation was performed under the conditions 1. As the organic substance, there were contained 26.0 mol% of trans-1,3,3,3-tetrafluoropropene (trans-TFP), 6.3 mol% of cis-1,3,3,3-tetrafluoropropene (cis-TEP1234zez), 17.7 mol% of 1,1,1,3,3-pentafluoropropane (PFP) and 49.6 mol% of 1-chloro-3,3,3-trifluoropropene (CTFP) in the inlet gas of the rough separation column 2, i.e., the reaction product A. Further, there were contained 55.6 mol% of trans-1,3,3,3-tetrafluoropropene (trans-TFP), 9.3 mol% of cis-1,3,3,3-tetrafluoropropene (cis-TEP), 9.6 mol% of 1,1,1,3,3-pentafluoropropane (PFP) and 25.3 mol% of 1-chloro-3,3,3-trifluoropropene (CTFP) as the organic substance in the outlet gas of the rough separation column 2, i.e., the residue B.

Namely, the amount of the organic substance distilled as the residue B from the rough separation column 2, which predominantly contained the trans-1,3,3,3-tetrafluoropropene, was about 50% of the organic substance in the reaction product A of the reaction step; and the amount of the hydrogen chloride distilled was about 90 mol% of the theoretical by-production amount when the distillation operation was performed by the rough separation column 2 under the conditions 1

As seen from these results, it was effective in the formation reaction of the trans-1,3,3,3-tetrafluoropropene (trans-TFP) from the 1-chloro-3,3,3-trifluoropropene (CTFP) and hydrogen fluoride (HF) to recover the organic substance containing the 1-chloro-3,3,3-trifluoropropene (CTFP) and 1,1,1,3,3-pentafluoropropane (PFP) and about 90 mol% of the hydrogen fluoride (HF) supplied to the reaction step, as the distillation bottom product *b* of the rough separation column 2 in the heating bath, and resupply the distillation bottom product *b* of the rough separation column 2 into the gas-phase reactor 1 of the reaction step for improvement of the reaction efficiency in the reaction step.

It was possible to easily supply the excessive amount of hydrogen fluoride (HF) relative to the reactant and obtain the trans-1,3,3,3-tetrafluoropropene (trans-TFP) with high selectivity by recovering the hydrogen fluoride (HF) by the rough separation column 2 and resupplying the recovered the hydrogen fluoride (HF) to the gas-phase reactor 1 of the reaction step. As the major portion of the hydrogen fluoride in the reaction product was recovered by the rough separation step 2, it was possible to provide a significant load reduction in the recovery of the hydrogen fluoride (HF) by treatment with the sulfuric acid by the hydrogen fluoride absorption column 3 and diffusion column 4 in the hydrogen fluoride separation step, the separation of the hydrogen chloride (G) by the hydrogen chloride absorption column 5 in the hydrogen chloride separation step and the purification of the trans-1,3,3,3-tetrafluoropropene (trans-TFP) by the rectification column 8 in the purification step.

### [Process Example 2]

The gas-phase reactor 1 of stainless steel (SUS316L), which had a cylindrical reaction tube of 52.7 cm in inside diameter and 58 cm in length, was again packed with 1200 ml of the fluorination catalyst of Catalyst Preparation Example 1.

Further, the rough separation column 2 of 54.9 mm in inside diameter and 40 cm in length, which had a cooling condenser at a top side thereof and a heating bath at a bottom side thereof, was also packed with 6 mm Raschig rings. The formation reaction of trans-1,3,3,3-tetrafluoropropene (trans-TFP) from 1-chloro-3,3,3-trifluoropropene (CTFP) and hydrogen fluoride (HF) was carried out in the gas-phase reactor 1 by varying the reaction conditions while distilling the reaction product under the same distillation conditions 1 by means of the rough separation column 2 as above and supplying the hydrogen fluoride (HF) and organic substance recovered as the distillation bottom product *b* of the rough separation column 2, together with newly added 1-chloro-3,3,3-trifluoropropene (CTFP) and hydrogen fluoride (HF), as the raw material to the gas-phase reactor 1.

The reaction conditions and results are indicated in TABLE 3. As mentioned above, the trans-1,3,3,3-tetrafluoropropene (trans-TFP) and hydrogen fluoride (HF) were newly supplied into the gas-phase reactor 1 while returning the organic substance recovered as the distillation bottom product *b* of the rough separation column 2 to the gas-phase reactor 1. Herein, the composition ratio of the recovered organic substance (the selectivity of the respective product components) was measured by GC-FID in units of mol%. In the recovered organic substance, there were contained 9.7 mol% of the trans-1,3,3,3-tetrafluoropropene (trans-TFP), 4.8 mol% of the cis-1,3,3,3-tetrafluoropropene (cis-TFP), 40.3 mol%of the 1,1,1,3,3-pentafluoropropane (PFP) and 41.1 mol% of the 1-chloro-3,3,3-trifluoropropene (CTFP).

**TABLE 3**

| | CTFP (g/min) | Recovered organic substance (g/min) | HF (g/min) | Pressure (MPa) | Temp. (°C) | Selectivity (mol%) | | | |
|---|---|---|---|---|---|---|---|---|---|
| | | | | | | Trans-TEP | Cis-TEP | PEP | CTFP |
| Preparation Example 1 | 1.2 | 2.7 | 3.5 | 0.1 | 360 | 34.4 | 7.5 | 17.3 | 40.8 |
| | 1.2 | 2.7 | 3.5 | 0.2 | 360 | 29.1 | 6.4 | 24.5 | 39.9 |
| | 1.2 | 2.7 | 7.1 | 0.1 | 380 | 47.3 | 8.3 | 12.4 | 32.0 |

As shown in TABLE 3, the reaction was carried out by setting the flow rate of the hydrogen fluoride (HF) to 3.5 g/min or 7.1 g/min and setting the reaction pressure to 0.1 MPa or 0.2 MPa while maintaining the reaction temperature, the flow rate of the 1-chloro-3,3,3-trifluoropropene (CTFP) and the flow rate of the recovered organic substance at 360°C, 1.2 g/min and 2.7 g/min, respectively. The selectivity of the trans-1,3,3,3-tetrafluoropropene (trans-TFP) was 34.4 mol% or 29.2 mol% when the flow rate of the hydrogen fluoride (HF) was 3.5 g/m and was 47.3 mol% when the flow rate of the hydrogen fluoride (HF) was 7.1 g/min. The selectivity of the trans-1,3,3,3-tetrafluoropropene (trans-TFP) was thus higher when the HF flow rate was 3.5 g/m than when the HF flow rate was 7.1 g/min.

### [Dehydrofluorination (Hydrogen Fluoride Separation Step), Dehydrochlorination (Hydrogen Chloride Separation Step) and Dehydration (Dehydration Drying Step]

The reaction product A was distilled by the rough separation column 2 under the same distillation conditions 2 as in the rough distillation step. The residue B extracted from the rough separation column 2 was brought into contact with sulfuric acid in the hydrogen fluoride separation absorption column 3 to remove hydrogen fluoride (HF) by absorption into the sulfuric acid in the hydrogen fluoride separation step.

The residue C of the hydrogen fluoride separation step was bubbled into water at a rate of 2.0 g/min within the hydrogen chloride absorption column 5 to remove hydrogen chloride *e* from the residue C.

In the subsequent dehydration drying step, the mist separator 6 of SUS316 was packed with a SUS316 packing material and cooled with a cooling medium of 5°C. The residue D remaining after the separation of the hydrogen fluoride *e* was then introduced into the mist separator 6 to separate and remove entrained water mist h1 from the residue D. After that, the residue D, i.e., mixed gas of organic compounds was collected at the outlet of the mist separator 6. The water content of the collected mixed gas was determined to be 1300 ppm according to the Karl Fischer's method.

### [Purification of Trans-1,3,3,3-tetrafluoropropene (Purification Step)]

In the subsequent purification step, a double-tube type cooling device of SUS316 of 12 mm in outer tube inside diameter, 6 mm in inner tube outside diameter and 300 mm in length was provided as the heat exchanger 7. The gaseous residue D discharged the mist separator 6 was cooled by supplying the residue D at a rate of 2.0 g/min into the space between the inner and outer tube of the double-tube type cooling device while flowing a cooling medium of -40°C through the inner tube. The thus-liquefied organic substance (residue E) was collected from the bottom of the cooling device. The water content of the collected organic substance was determined to be 65 ppm according to the Karl Fischer's method. No organic component was found by GC analysis of the organic substance. As a result, 98 mass% of the organic substance introduced into the cooling device was recovered.

The above dehydrated organic substance, i.e., residue E was distilled by the rectification column 8 to isolate the trans-1,3,3,3-tetrafluoropropene (trans-TEP) as the distillate in the purification step. The water content of the isolated trans-1,3,3,3-tetrafluoropropene (trans-TEP) was determined to be 78 ppm according to the Karl Fischer's method; and the purity was determined by gas chromatography to be 99.9%.

As seen from the above examples, it was possible to not only reduce the amount of sulfuric acid brought into contact with the residue B to remove the hydrogen fluoride in the hydrogen fluoride separation step for easy plant operation, but also ease the separation of the hydrogen chloride in the hydrogen chloride separation step and the dehydration operation of the dehydration drying step, when the distillation operation was performed in the rough separation step to recover the unreacted 1-chloro-3,3,3-trifluoropropene and the major portion of the hydrogen fluoride as the distillation bottom product in the production process of the trans-1,3,3,3-tetrafluoropropene according to the present invention. It is also apparent that it was possible in actual production to secure saving in operation labor, operation stability and safety in the rough separation step and its subsequent steps as well as equipment protection against the hydrofluoric acid. It was further possible, when the rough separation step was performed, to easily obtain the trans-1,3,3,3-tetrafluoropropene with high purity by the distillation purification of the reaction product in the purification step.

Although the present invention has been described with reference to the above specific embodiments, the present invention is not limited to these exemplary embodiments. Various modifications and variations of the embodiments described above can be made based on the general knowledge of those skilled in the art without departing from the scope of the present invention.

### Description of Reference Numerals

1: Gas-phase reactor
2: Rough separation column
3: Hydrogen fluoride absorption column
4: Diffusion column
5: Hydrogen chloride absorption column 5
6: Mist separator
7: Heat exchanger
8: Rectification column
A: Reaction product
B to E: Residue (1,3,3,3-tetrafluoropropene, 1-chloro-3,3,3-trifluoropropene, 1,1,1,3,3-pentafluoropropane and the like)
F: Residue (distillation residue)
a: Raw material (1-chloro-3,3,3-trifluoropropene and hydrogen fluoride)
b: Distillation bottom product (recovered organic substance such as unreacted 1-chloro-3,3,3-trifluoropropene and hydrogen fluoride)
c: Hydrogen fluoride and sulfuric acid
d: Hydrogen fluoride
e: Hydrochloric acid
h1, h2: Water content
g: Trans-1,3,3,3-tetrafluoropropene

## Claims

1. A production process of trans-1,3,3,3-tetrafluoropropene, comprising:
a reaction step of reacting 1-chloro-3,3,3-trifluoropropene with hydrogen fluoride to form trans-1,3,3,3-tetrafluoropropene and obtain a reaction product A containing the formed trans-1,3,3,3-tetrafluoropropene, unreacted 1-chloro-3,3,3-trifloropropene and hydrogen fluoride and by-produced cis-1,3,3,3-tetrafluoropropene, 1,1,1,3,3-pentafluoropropane and hydrogen chloride;
a rough separation step of distilling the reaction product A obtained in the reaction step to recover a distillation bottom product containing the 1-chloro-3,3,3-trifloropropene and hydrogen fluoride, and then, supplying the recovered distillation bottom product to the reaction step;
a hydrogen fluoride separation step of recovering the hydrogen fluoride from a residue B remaining after the recovery of the distillation bottom product in the rough separation step and supplying the recovered hydrogen fluoride to the reaction step;
a hydrogen chloride separation step of bringing a residue C remaining after the recovery of the hydrogen fluoride in the hydrogen fluoride separation step into contact with water or an aqueous sodium hydroxide solution to thereby separate the hydrogen chloride;
a dehydration drying step of dehydrating a residue D remaining after the separation of the hydrogen chloride in the hydrogen chloride separation step; and
a purification step of obtaining the trans-1,3,3,3-tetrafluoropropene by distillation of a residue E remaining after the dehydration in the dehydration drying step.

2. The production process according to claim 1, wherein, in the reaction step, the trans-1,3,3,3-tetrafluoropropene is formed by fluorination of the 1-chloro-3,3,3-trifluoropropene with the hydrogen chloride in a gas phase in the presence of a fluorination catalyst.

3. The production process according to claim 2, wherein, in the reaction step, the fluorination is performed in the gas phase under the conditions of a pressure of 0.05 to 0.3 MPa and a temperature of 200 to 450°C.

4. The production process according to claim 2 or 3, wherein the fluorination catalyst is either a nitrate, a chloride, an oxide, a sulfate, a fluoride, a fluorochloride, an oxyfluoride, an oxychloride or an oxyfluorochloride of at least one kind of metal selected from the group consisting of chromium, titanium, aluminum, manganese, nickel, cobalt, titanium, iron, copper, zinc, silver, molybdenum, zirconium, niobium, tantalum, iridium, tin, hafnium, vanadium, magnesium, lithium, sodium, potassium, calcium and antimony.

5. The production process according to any one of claims 1 to 3, wherein, in the reaction step, the fluorination is performed in the gas phase in the presence of chromium chloride supported on fluorinated alumina as the fluorination catalyst under the conditions of a pressure of 0.05 to 0.3 MPa and a temperature of 350 to 450°C by the supply of the 1-chloro-3,3,3-trifluoropropene and hydrogen fluoride at a mole ratio of 1-chloro-3,3,3-trifluoropropene:hydrogen fluoride = 1:8 to 1:25.

6. The production process according to any one of claims 1 to 3, wherein, in the reaction step, the fluorination is performed in the gas phase in the presence of either an oxide, a fluoride, a chloride, a fluorochloride, an oxyfluoride, an oxychloride or an oxyfluorochloride of chlromium supported on activated carbon as the fluorination catalyst under the conditions of a pressure of 0.05 to 0.3 MPa and a temperature of 350 to 450°C by the supply of the 1-chloro-3,3,3-trifluoropropene and hydrogen fluoride at a mole ratio of 1-chloro-3,3,3-trifluoropropene:hydrogen fluoride = 1:8 to 1:25.

7. The production process according to any one of claims 1 to 6, wherein, in the hydrogen fluoride separation step, the hydrogen fluoride is recovered by absorption into sulfuric acid.

8. The production process according to any one of claims 1 to 7, wherein, in the dehydration drying step, the residue D remaining after the hydrogen chloride separation step is dehydrated by freezing and solidifying water contained in the residue D by means of a heat exchanger.

9. The production process according to any one of claims 1 to 7, wherein, in the dehydration drying step, the residue D remaining after the hydrogen chloride separation step is dehydrated by adsorption of water contained in the residue D onto an adsorbent.

10. The production process according to any one of claims 1 to 9, further comprising a step of supplying a distillation residue F remaining after the purification step to the reaction step.

11. The production process according to claim 10, wherein the distillation residue F remaining after the purification step is supplied to the reaction step after converting the cis-1,3,3,3-tetrafluoropropene contained in the distillation residue F to 1,1,1,3,3-pentafluoropropane.
